(19) Europäisches Patentamt
European Patent Office
Office européen des brevets

(11) **EP 3 832 292 B1**

(12) **EUROPEAN PATENT SPECIFICATION**

(45) Date of publication and mention
of the grant of the patent:
**30.08.2023 Bulletin 2023/35**

(21) Application number: **19844087.7**

(22) Date of filing: **31.07.2019**

(51) International Patent Classification (IPC):
**G01N 21/51** *(2006.01)*      **C12M 1/34** *(2006.01)*
**G01N 21/03** *(2006.01)*      **G01N 33/579** *(2006.01)*
**G01N 33/92** *(2006.01)*      **G01N 21/77** *(2006.01)*
**G01N 33/543** *(2006.01)*      **G01N 33/549** *(2006.01)*
**G01N 21/41** *(2006.01)*

(52) Cooperative Patent Classification (CPC):
**G01N 21/41; G01N 21/7743; G01N 33/54366;
G01N 33/54373; G01N 33/549; G01N 33/579;**
G01N 2021/4126; G01N 2021/4166

(86) International application number:
**PCT/JP2019/030010**

(87) International publication number:
**WO 2020/027197 (06.02.2020 Gazette 2020/06)**

(54) **INSPECTING METHOD, INSPECTING INSTRUMENT, AND INSPECTING DEVICE**

INSPEKTIONSVERFAHREN, INSPEKTIONSINSTRUMENT UND INSPEKTIONSVORRICHTUNG

PROCÉDÉ D'INSPECTION, INSTRUMENT D'INSPECTION ET DISPOSITIF D'INSPECTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.07.2018 JP 2018143726
20.03.2019 JP 2019052598**

(43) Date of publication of application:
**09.06.2021 Bulletin 2021/23**

(73) Proprietors:
• **SEKISUI CHEMICAL CO., LTD.
Osaka-shi
Osaka
530-8565 (JP)**
• **University Public Corporation Osaka
Osaka-shi, Osaka 545-0051 (JP)**

(72) Inventors:
• **AKAGI, Yoshinori
Mishima-gun, Osaka 618-0021 (JP)**
• **ASANO, Motohiko
Mishima-gun, Osaka 618-0021 (JP)**
• **DOI, Jun
Mishima-gun, Osaka 618-0021 (JP)**

• **ENDO, Tatsuro
Sakai-shi, Osaka 599-8531 (JP)**

(74) Representative: **Ter Meer Steinmeister & Partner
Patentanwälte mbB
Nymphenburger Straße 4
80335 München (DE)**

(56) References cited:
**WO-A1-2017/138595      WO-A1-2017/138595
JP-A- H04 152 249      JP-A- 2002 122 601
JP-A- 2005 321 196      JP-A- 2005 516 210
US-A1- 2006 193 550      US-A1- 2006 286 663
US-A1- 2008 240 543      US-A1- 2009 079 976
US-A1- 2009 153 845      US-A1- 2015 268 237**

• **ARMANDO RICCIARDI ET AL: "Broadband
Mirrors in the Near-Infrared Based on
Subwavelength Gratings in SOI", IEEE
PHOTONICS JOURNAL, IEEE, USA, vol. 2, no. 5,
1 October 2010 (2010-10-01) , pages 696-702,
XP011484995, ISSN: 1943-0655, DOI:
10.1109/JPHOT.2010.2059003**

EP 3 832 292 B1

- **TABASSUM SHAWANA ET AL: "Nanopatterned Optical Fiber Tip for Guided Mode Resonance and Application to Gas Sensing", IEEE SENSORS JOURNAL, IEEE, USA, vol. 17, no. 22, 15 November 2017 (2017-11-15), pages 7262-7272, XP011672286, ISSN: 1530-437X, DOI: 10.1109/JSEN.2017.2748593 [retrieved on 2017-10-23]**

**Description**

**TECHNICAL FIELD**

The present invention relates to an inspecting method for measuring the concentration of a chemical substance. Further, the present invention relates to an inspecting instrument and an inspecting device that are used for measuring the concentration of a chemical substance.

**BACKGROUND ART**

A method for measuring the concentration of a biological material such as endotoxin has been known.

For example, Patent Document 1 below discloses a method for measuring the concentration of endotoxin by measuring, according to an interference enhanced reflection method (IER method), the film thickness of a gel film formed through a reaction of LAL reagent with endotoxin.

Patent Document 2 below discloses a method for measuring the concentration of endotoxin or β-D-glucan. In this inspecting method, a sample solution that contains a coagulin monomer or an aggregate thereof produced through a reaction of LAL reagent with endotoxin or β-D-glucan is irradiated with light, and the intensity of the resulting scattered light is measured to measure the concentration of the endotoxin or the β-D-glucan.

Patent Document 3 below discloses an inspecting instrument used for measuring the concentration of a test substance. This inspecting instrument includes a compound capable of producing a granular substance through a reaction with the test substance, or a compound that is capable of being bound with the test substance and is a granular substance. The inspecting instrument also includes a wall having a periodic structure on a surface thereof. In Patent Document 3, the concentration of the test substance is measured by introducing a test substance-containing solution containing the test substance and water into the inspecting instrument, removing the water, and then irradiating a surface of the periodic structure with light.

Related Art Documents

**Patent Documents**

Patent Document 1: JP 2007-327946 A
Patent Document 2: JP 2010-032436 A
Patent Document 3: WO 2017/138595 A1.

US 2009/079976 A1 discloses a biosensor for measuring a test substance in a solution such as detecting an antibody with an immobilized ligand. The biosensor comprises a photonic crystal sensor comprising a grating structure defined by a plurality of alternating high and low regions of the grating structure as well as a thin film 180 of a relatively high index of refraction material such as $TiO_2$ or $Ti_2O_3$ layer deposited onto the grating structure.

**SUMMARY OF THE INVENTION**

**PROBLEMS TO BE SOLVED BY THE INVENTION**

In recent years, the concentration of a biological material such as endotoxin is being required to be measured with much higher accuracy. The conventional methods described in Patent Documents 1 and 2, however, have difficulty measuring the concentration of endotoxin at, for example, 0.001 EU/ml or less with high accuracy.

The inspecting instrument described in Patent Document 3 is capable of measuring the concentration of a biological material with high accuracy. For example, in the examples of Patent Document 3, the concentration of endotoxin at around 0.001 EU/ml and the concentration of a test substance such as a protein at around 10 pg/ml are measured with high accuracy. However, the concentration of a test substance at a concentration further lower than the concentrations of the test substances measured in the examples of Patent Document 3 is being required to be measured with high accuracy.

In Patent Document 3, the water is removed after the test substance-containing solution containing the test substance and the water is introduced into the inspecting instrument. Then, in order to measure the concentration of the test substance while the water has been removed, the surface of the periodic structure in the inspecting instrument is irradiated with light. Such a measuring method requires an operation for removing a liquid such as water and prolongs the time for obtaining a measurement result.

The inventors of the present invention have studied about an inspecting instrument capable of measuring the concentration of a test substance with high accuracy while a test substance-containing solution containing the test substance and a liquid are present. The inventors of the present invention have found that when the concentration of the test substance is significantly low (for example, a test substance such as a protein at a level of fg/ml), the inspecting instrument described in Patent Document 3 has difficulty measuring the concentration of the test substance in the liquid and measuring the concentration of the test substance with high accuracy.

An object of the present invention is to provide an inspecting method and an inspecting instrument that are capable of measuring the concentration of a test substance in a liquid and measuring the concentration of a test substance with high accuracy. Another object of the present invention is to provide an inspecting method using the inspecting instrument.

## MEANS FOR SOLVING THE PROBLEMS

According to a broad aspect of the present invention, provided is an inspecting method for measuring, using a test substance-containing solution containing a test substance and a liquid, a concentration of the test substance, which is contained in the test substance-containing solution, in the liquid, the inspecting method using an inspecting instrument, the inspecting instrument including a wall that has a periodic structure resulting from a plurality of recesses or a plurality of protrusions, the plurality of recesses or the plurality of protrusions including a refractive index adjusting layer on surfaces thereof, the refractive index adjusting layer being a silicon layer, the inspecting method including the steps of: measuring a reference light intensity by irradiating the periodic structure with light while the liquid has been introduced into the inspecting instrument; measuring an evaluation light intensity by irradiating the periodic structure with light while the test substance-containing solution has been introduced into the inspecting instrument and the test substance and the liquid are present; and acquiring a relative value between the reference light intensity and the evaluation light intensity, and the inspecting method comparing the relative value obtained when a specimen with a known concentration of the test substance is introduced into the inspecting instrument, to the relative value obtained when a specimen with an unknown concentration of the test substance is introduced into the inspecting instrument, to determine the concentration of the test substance in the specimen with the unknown concentration of the test substance.

In a particular aspect of the inspecting method according to the present invention, the inspecting instrument includes a compound capable of producing a granular substance through a reaction with the test substance, or a compound that is capable of being bound with the test substance and is a granular substance, and in the step of measuring the evaluation light intensity, the evaluation light intensity is measured by irradiating the periodic structure with light while the test substance-containing solution has been introduced into the inspecting instrument and the granular substance is deposited on the refractive index adjusting layer.

In the inspecting method according to the present invention, the refractive index adjusting layer is a silicon layer.

In a particular aspect of the inspecting method according to the present invention, the silicon layer is a monocrystalline silicon layer, a polycrystalline silicon layer, a microcrystalline silicon layer, or an amorphous silicon layer.

In a particular aspect of the inspecting method according to the present invention, the silicon layer is the amorphous silicon layer.

In a particular aspect of the inspecting method according to the present invention, the test substance-containing solution is a solution containing a biological sample.

According to a broad aspect of the present invention, provided is an inspecting instrument used for measuring, using a test substance-containing solution containing a test substance and a liquid, a concentration of the test substance, which is contained in the test substance-containing solution, in the liquid, the inspecting instrument including a wall that has a periodic structure resulting from a plurality of recesses or a plurality of protrusions, the plurality of recesses or the plurality of protrusions including a refractive index adjusting layer on surfaces thereof, and the refractive index adjusting layer being a silicon layer.

In a particular aspect of the inspecting instrument according to the present invention, the refractive index adjusting layer contains a metallic element.

According to the present invention, the refractive index adjusting layer is a silicon layer.

In a particular aspect of the inspecting instrument according to the present invention, the silicon layer is a monocrystalline silicon layer, a polycrystalline silicon layer, a microcrystalline silicon layer, or an amorphous silicon layer.

In a particular aspect of the inspecting instrument according to the present invention, the silicon layer is the amorphous silicon layer.

In a particular aspect of the inspecting instrument according to the present invention, the refractive index adjusting layer has an average thickness of 1 nm or more and 100 nm or less.

In a particular aspect of the inspecting instrument according to the present invention, the inspecting instrument includes a compound capable of producing a granular substance through a reaction with the test substance, or a compound that is capable of being bound with the test substance and is a granular substance.

In a particular aspect of the inspecting instrument according to the present invention, the compound is not in contact with the refractive index adjusting layer and is disposed at a position away from the refractive index adjusting layer.

In a particular aspect of the inspecting instrument according to the present invention, the compound is disposed on a surface of the refractive index adjusting layer.

In a particular aspect of the inspecting instrument according to the present invention, when the inspecting instrument includes the compound capable of producing a granular substance through a reaction with the test substance, the compound capable of producing a granular substance through a reaction with the test substance is a compound capable of producing a granular substance in aggregation through a reaction with the test substance, and when the inspecting instrument includes the compound that is capable of being bound with the test substance and is a granular substance, the compound that is capable of being bound with the test substance and is a granular substance is a compound that is bound with the test substance and is thus capable of producing a granular substance in aggregation.

In a particular aspect of the inspecting instrument according to the present invention, the inspecting instrument includes the compound that is capable of being bound with the test substance and is a granular substance, the test substance contains an antigen, and the compound contains an antibody.

In a particular aspect of the inspecting instrument according to the present invention, the inspecting instrument includes the compound capable of producing a granular substance through a reaction with the test substance, the test substance is glycolipid, and the compound is an enzyme capable of being reacted with the glycolipid.

In a particular aspect of the inspecting instrument according to the invention, the inspecting instrument includes the compound capable of producing a granular substance through a reaction with the test substance, the test substance is endotoxin, and the compound is LAL reagent.

In a particular aspect of the inspecting instrument according to the present invention, the test substance-containing solution is a solution containing a biological sample.

In a particular aspect of the inspecting instrument according to the present invention, the inspecting instrument is a microchip.

According to a broad aspect of the present invention, provided is an inspecting device including the inspecting instrument described above, a light source for irradiating the periodic structure in the inspecting instrument with light, and a measuring device for measuring an intensity of light emitted from the light source and reflected on the periodic structure.

## EFFECTS OF THE INVENTION

An inspecting method according to the present invention is an inspecting method for measuring, using a test substance-containing solution containing a test substance and a liquid, a concentration of the test substance, which is contained in the test substance-containing solution, in the liquid. The inspecting method according to the present invention is an inspecting method using an inspecting instrument. In the inspecting method according to the present invention, the inspecting instrument includes a wall that has a periodic structure resulting from a plurality of recesses or a plurality of protrusions, and the plurality of recesses or the plurality of protrusions include a refractive index adjusting layer on surfaces thereof. In the inspecting method according to the present invention, the refractive index adjusting layer is a silicon layer. The inspecting method according to the present invention includes a step of measuring a reference light intensity by irradiating the periodic structure with light while the liquid has been introduced into the inspecting instrument. The inspecting method according to the present invention includes a step of measuring an evaluation light intensity by irradiating the periodic structure with light while the test substance-containing solution has been introduced into the inspecting instrument and the test substance and the liquid are present. The inspecting method according to the present invention includes a step of acquiring a relative value between the reference light intensity and the evaluation light intensity. In the inspecting method according to the present invention, the relative value obtained when a specimen with a known concentration of the test substance is introduced into the inspecting instrument is compared to the relative value obtained when a specimen with an unknown concentration of the test substance is introduced into the inspecting instrument, to determine the concentration of the test substance in the specimen with the unknown concentration of the test substance. The inspecting method according to the present invention configured as described above is capable of measuring the concentration of the test substance in the liquid and measuring the concentration of the test substance with high accuracy.

An inspecting instrument according to the present invention is an inspecting instrument used for measuring, using a test substance-containing solution containing a test substance and a liquid, a concentration of the test substance, which is contained in the test substance-containing solution, in the liquid. The inspecting instrument according to the present invention includes a wall that has a periodic structure resulting from a plurality of recesses or a plurality of protrusions, and the plurality of recesses or the plurality of protrusions include a refractive index adjusting layer on surfaces thereof. In the inspecting instrument according to the present invention, the refractive index adjusting layer is a layer having a refractive index greater than a refractive index of the test substance-containing solution or is a silicon layer. The inspecting instrument according to the present invention configured as described above is capable of measuring the concentration of the test substance in the liquid and measuring the concentration of the test substance with high accuracy.

## BRIEF DESCRIPTION OF DRAWINGS

[Fig. 1] Fig. 1 is a front sectional view of an inspecting instrument according to a first embodiment of the present invention.

[Fig. 2] Fig. 2 is a plan view of the inspecting instrument according to the first embodiment of the present invention.

[Fig. 3] Fig. 3 is an enlarged front sectional view of an inspecting instrument according to a second embodiment of the present invention.

[Fig. 4] Fig. 4 is a front sectional view of an inspecting instrument according to a third embodiment of the present invention.

[Fig. 5] Fig. 5 is a plan view of the inspecting instrument according to the third embodiment of the present invention.

[Fig. 6] Fig. 6 is an enlarged front sectional view of an inspecting instrument according to a fourth embodiment of the present invention.

[Fig. 7] Fig. 7 is a front sectional view for describing one example of an inspecting method using the inspecting instrument according to the first embodiment of the present invention.

[Fig. 8] Fig. 8 is an enlarged front sectional view for describing the one example of the inspecting method using the inspecting instrument according to the first embodiment of the present invention.

[Fig. 9] Fig. 9 is an enlarged front sectional view for describing the one example of the inspecting method using the inspecting instrument according to the first embodiment of the present invention.

[Fig. 10] Fig. 10 is an enlarged front sectional view for describing one example of the inspecting method using the inspecting instrument according to the first embodiment of the present invention.

[Fig. 11] Fig.11 is an enlarged front sectional view for describing one example of an inspecting method using the inspecting instrument according to the fourth embodiment of the present invention.

[Fig. 12] Fig. 12 is an enlarged front sectional view for describing a modified example of the inspecting method illustrated in Fig. 11.

[Fig. 13] Fig. 13 is a schematic view of an inspecting device according to the first embodiment of the present invention.

[Fig. 14] Fig. 14 is a schematic view of an inspecting device according to the second embodiment of the present invention.

[Fig. 15] Fig. 15 is a graph illustrating a relationship between the concentration of cystatin C and the wavelength shift between reflection spectra in Example 1 and Comparative Example 1.

[Fig. 16] Fig. 16 is a graph illustrating a relationship among the average thickness of a refractive index adjusting layer, the wavelength of a spectrum, and the reflected light intensity in Examples 3 and 4 and Comparative Example 2.

[Fig. 17] Fig. 17 is a graph illustrating a relationship between the concentration of cystatin C and the wavelength shift between reflection spectra in Example 5.

[Fig. 18] Fig. 18 is a graph illustrating a relationship, which is obtained by simulations, between the radius of protrusions or recesses in a periodic structure of a wall and the wavelength of a photonic band gap of the periodic structure of the wall.

[Fig. 19] Fig. 19 is a graph illustrating a relationship, which is obtained by simulations, between the radius of protrusions in periodic structures of walls and the inclination of a plurality of approximate straight lines.

[Fig. 20] Fig. 20 is a graph illustrating a relationship, which is obtained by simulations, between the radius of protrusions in periodic structures of walls and the inclination of a plurality of approximate straight lines.

## MODES FOR CARRYING OUT THE INVENTION

Hereinafter, the present invention is described in detail.

An inspecting method according to the present invention is an inspecting method for measuring, using a test substance-containing solution containing a test substance and a liquid, a concentration of the test substance, which is contained in the test substance-containing solution, in the liquid. The inspecting method according to the present invention is an inspecting method using an inspecting instrument. In the inspecting method according to the present invention, the inspecting instrument includes a wall that has a periodic structure resulting from a plurality of recesses or a plurality of protrusions, and the plurality of recesses or the plurality of protrusions include a refractive index adjusting layer on surfaces thereof. Accordingly, the wall includes the refractive index adjusting layer. In the inspecting method according to the present invention, the refractive index adjusting layer is silicon layer. The inspecting method according to the present invention includes a step of measuring a reference light intensity by irradiating the periodic structure with light while the liquid has been introduced into the inspecting instrument. The inspecting method according to the present invention includes a step of measuring an evaluation light intensity by irradiating the periodic structure with light while the test substance-containing solution has been introduced into the inspecting instrument and the test substance and the liquid are present. The inspecting method according to the present invention includes a step of acquiring a relative value between the reference light intensity and the evaluation light intensity. In the inspecting method according to the present invention, the relative value obtained when a specimen with a known concentration of the test substance is introduced into the inspecting instrument is compared to the relative value obtained when a specimen with an unknown concentration of the test substance is introduced into the inspecting instrument, to determine the concentration of the test substance in the specimen with the unknown concentration of the test substance.

An inspecting instrument according to the present invention is an inspecting instrument used for measuring, using a test substance-containing solution containing a test substance and a liquid, a concentration of the test substance, which is contained in the test substance-containing solution, in the liquid. The inspecting instrument according to the present invention includes a wall that has a periodic structure resulting from a plurality of recesses or a plurality of protrusions, and the plurality of recesses or the plurality of protrusions include a refractive index adjusting layer on surfaces thereof. Accordingly, the wall includes the refractive index adjusting layer. In the inspecting instrument according to the present invention, the refractive index adjusting layer is a layer having a refractive index greater than a refractive index of the test substance-containing solution or is a silicon layer.

The inspecting instrument and the inspecting method according to the present invention that are configured as described above are capable of measuring the concentration of the test substance in the liquid and measuring the concentration of the test substance with high accuracy. The inspecting instrument and the inspecting method according to the present invention that do not require removal of the liquid before the measurement enable rapid acquisition of a measurement result and an increase of measurement efficiency. Further, the inspecting instrument and the inspecting method according to the present invention that are configured as described above are capable of measuring the concentration of the test substance with high accuracy even when the concentration of the test substance is significantly low.

The inspecting instrument and the inspecting method according to the present invention that are capable of measuring the concentration of the test substance in the liquid enable a simple inspection to increase convenience.

The inventors of the present invention have, as the method for measuring the concentration of a test substance, studied about a method for introducing a specimen containing a test substance into an inspecting instrument, irradiating the inspecting instrument with light, and obtaining the concentration of the test substance from the intensity of the resultant reflected light. The use of a conventional inspecting instrument in this method causes a problem of making it difficult to measure the concentration of the test substance in the liquid or to measure the concentration of the test substance with high accuracy when the concentration of the test substance is significantly low (for example, a test substance such as a protein at a level of fg/ml).

The inventors of the present invention have found that a reason for making it difficult to measure the concentration of the test substance in the liquid or to measure the concentration of the test substance with high accuracy particularly when the concentration of the test substance is significantly low is a small difference in the refractive index between the inspecting instrument and the test substance-containing solution. As a material for the inspecting instrument, a resin having a refractive index of about 1.5 or glass having a refractive index of about 1.4 is generally used. On the other hand, the test substance-containing solution generally has a refractive index of about 1.3. The inventors of the present invention have found that a difference in the refractive index between the inspecting instrument and the test substance-containing solution of about 0.1 to 0.2 makes it difficult to measure the concentration of the test substance with high accuracy when the concentration of the test substance is significantly low.

The present invention configured as described above is capable of measuring the concentration of the test substance in the liquid, and measuring the concentration of the test substance with high accuracy even when the concentration of the test substance is significantly low. Further, the inspecting instrument and the inspecting method according to the present invention are capable of measuring the concentration of the test substance with high accuracy also when the concentration of the test substance is not low.

(Inspecting instrument)

The inspecting instrument according to the present invention includes a wall that has a periodic structure resulting from a plurality of recesses or a plurality of protrusions, and the plurality of recesses or the plurality of protrusions include a refractive index adjusting layer on surfaces thereof. In the inspecting instrument according to the present invention, the refractive index adjusting layer is a layer having a refractive index greater than a refractive index of the test substance-containing solution or is a silicon layer. The refractive index adjusting layer may be the layer having a refractive index greater than the refractive index of the test substance-containing solution or may be the silicon layer.

The wall has the periodic structure. The periodic structure of the wall includes the plurality of recesses or the plurality of protrusions. The periodic structure of the wall is the periodic structure resulting from the plurality of recesses or the plurality of protrusions.

In the present specification, a wall excluding the refractive index adjusting layer is described as a "wall X" to distinguish the wall excluding the refractive index adjusting layer from the wall including the refractive index adjusting layer. The inspecting instrument according to the present invention includes the wall X having, on a surface thereof, a periodic structure resulting from a plurality of recesses or a plurality of protrusions and includes the refractive index adjusting layer disposed on a surface of the periodic structure of the wall X. The wall including the refractive index adjusting layer is a composite wall including the wall X and the refractive index adjusting layer. In the description below, the wall described as the "wall" means the wall including the refractive index adjusting layer. In the description below, the wall described as the "wall X" means the wall excluding the refractive index adjusting layer.

From the viewpoint of measuring the concentration of the test substance with much higher accuracy, the periodic structure of the wall has a period of preferably 100 nm or more, more preferably 200 nm or more, further preferably 250 nm or more, and preferably 800 nm or less, more preferably 500 nm or less, further preferably 450 nm or less.

The period of the periodic structure of the wall means a gap between the tip (a central portion when the tip is a flat or the like) of a protrusion or a recess and the tip (a central portion when the tip is a flat) of a protrusion or a recess most adjacent to the starting-point protrusion or recess.

From the viewpoint of measuring the concentration of the test substance with much higher accuracy, the recesses or the protrusions in the periodic structure of the wall have a radius of preferably 20 nm or more, more preferably 30 nm or more, further preferably 40 nm or more, and preferably 300 nm or less, more preferably 200 nm or less, further preferably 150 nm or less.

The radius of the recesses or the protrusions in the periodic structure of the wall means the radius of a circle when the shape of the upper surfaces of the recesses or the protrusions is circle, and means, when the shape of the upper surfaces of the recesses or the protrusions is a shape other than circle, the radius of an inscribed circle of the shape other than circle.

The refractive index adjusting layer is disposed on the surface of the periodic structure of the wall X. The refractive index adjusting layer may be disposed on the entire surface of the periodic structure of the wall X or may be disposed on a part of the surface of the periodic structure of the wall X. The refractive index adjusting layer need not be disposed on side surfaces of the recesses or the protrusions in the periodic structure of the wall X. The disposition of the refractive index adjusting layer on a part of the surface or the entire surface of the periodic structure of the wall X enables a periodic structure corresponding to the periodic structure of the wall X to be easily formed on the surface of the refractive index adjusting layer.

From the view point of measuring the concentration of the test substance with much higher accuracy, the periodic structure of the wall X has a surface area on which the refractive index adjusting layer is disposed of preferably 60% or more, more preferably 70% or more, further preferably 80% or more, particularly preferably 90% or more, most preferably 100%, in 100% of the surface area (the plurality of recesses or the plurality of protrusions) of the periodic structure. The refractive index adjusting layer is most preferably disposed on the entire surface of the periodic structure of the wall X. The refractive index adjusting layer preferably also has the periodic structure resulting from the plurality of recesses or the plurality of protrusions.

In the inspecting instrument according to the present invention, the period of the periodic structure of the wall, the radius of the plurality of recesses or the plurality of protrusions, and the refractive index of the periodic structure are preferably adjusted so that the value that can be obtained as a wavelength $\lambda$ of diffracted light in the periodic structure of the wall becomes 300 nm or more and 1600 nm or less when m is any positive integer in the following equations (1) and (2). In this case, a generally easily available light source can be used.

[Equation 1]

$$\lambda = \frac{2d\sqrt{(n_0)^2 - (\sin\theta)^2}}{m} \quad (1)$$

[Equation 2]

$$n_0 = (1-f)n_1 - fn_2 \quad (2)$$

In the equation (1) or (2), m represents the degree, $\lambda$ represents the wavelength (nm) of the diffracted light, d represents the period (nm) of the periodic structure of the wall, $n_0$ represents an effective refractive index, $\theta$ represents an incident angle of light, f represents porosity, that is, occupancy of voids per unit volume, $n_1$ represents a refractive index of the periodic structure of the wall, and $n_2$ represents a refractive index of a porous material.

The refractive index $n_1$ of the periodic structure of the wall refers to a refractive index at a portion including no porous portion but including the refractive index adjusting layer in the periodic structure of the wall.

From the viewpoint of measuring the concentration of the test substance with much higher accuracy, the degree m is preferably 1, 2, or 3, more preferably 1 or 2, further preferably 1 in the equation (1).

The wavelength $\lambda$ of the diffracted light obtained by the equations (1) and (2) is preferably 600 nm or more, more preferably 700 nm or more, and preferably 1200 nm or less, more preferably 1000 nm or less.

The inspecting instrument according to the present invention preferably includes a compound (first compound) capable of producing a granular substance through a reaction with the test substance, or a compound (second compound) that is capable of being bound with the test substance and is a granular substance. In this case, the inspecting instrument may include the first compound or may include the second compound.

A principle of measuring the concentration of the test substance using the inspecting instrument is described below.

The inspecting instrument is capable of reacting or binding the test substance with the compound (the first compound or the second compound). The granular substance produced through the reaction of the test substance with the compound or the granular substance in which the test substance is bound to the compound can be deposited on the refractive index adjusting layer (on the periodic structure of the wall). This deposition can be progressed in the liquid.

The deposition of the granular substance on the periodic structure of the wall changes the refractive index $n_1$ of the periodic structure of the wall to change the wavelength $\lambda$ of the diffracted light attributed to the periodic structure of the wall. Accordingly, the concentration of the test substance can be measured by detecting a change in the spectrum of the reflected light when the periodic structure of the wall is irradiated with light.

The inspecting instrument according to the present invention configured as described above is capable of enormously increasing the amount of the change in the refractive index $n_1$ of the periodic structure of the wall between before and after the deposition of the granular substance. That is, only a slight amount of the granular substance deposited on the periodic structure of the wall enables the spectrum of the reflected light to be changed to a detectable degree. Accordingly, the accuracy of measuring the concentration of the test substance can be dramatically increased.

The granular substance produced through the reaction of the test substance with the compound or the granular substance in which the test substance is bound to the compound may be a granular substance in aggregation or need not be a granular substance in aggregation. Conventionally, sometimes used is a method for measuring the concentration of the test substance by detecting light scattering caused by an aggregate. This method, however, is considered to have had difficulty attaining the measurement when the concentration is particularly low. That is, it is considered that the detection cannot be attained until the aggregate becomes a certain size (for example, a micrometer order) or the accuracy is low due to the variation in the degree of the aggregation. In the present invention, slight presence of the granular substance such as a reaction product of the test substance and the compound on a surface end of the fine periodic structure (for example, a nanometer order) has been found to change an optical response (for example, reflected light intensity) from the surface of the periodic structure of the wall (the surface including the refractive index adjusting layer disposed on the surface of the periodic structure of the wall X) . Therefore, the present invention is capable of measuring the concentration of the test substance even at a low concentration with much higher accuracy when the granular substance is not a granular substance in aggregation. Further, the concentration can be measured more promptly than in conventional methods.

The compound is neither in contact with the periodic structure of the wall nor the refractive index adjusting layer, and may be disposed at a position away from the periodic structure of the wall and from the refractive index adjusting layer. In this case, at the position away from the periodic structure of the wall and from the refractive index adjusting layer, the test substance can be reacted or bound with the compound. The granular substance can be more securely deposited on the refractive index adjusting layer by gravity or the like. Alternatively, the compound may be disposed on the surface of the refractive index adjusting layer. In this case, the change of the optical response from the refractive index adjusting layer can be more promptly detected.

As the compound capable of producing a granular substance through a reaction with the test substance, for example, a compound that forms a gel through a reaction with the test substance can be used.

Hereinafter, specific embodiments of the present invention are described with reference to drawings.

The drawings referred to in the embodiments are illustrated schematically, and the dimensional ratio and the like of objects depicted in the drawings are sometimes different from those of actual objects. The specific dimensional ratio and the like of objects should be determined with the following description taken into consideration. Particularly, in the drawings referred to, the size of recesses is shown in an enlarged manner for convenience of illustration. Actual recesses are nano-sized and finer.

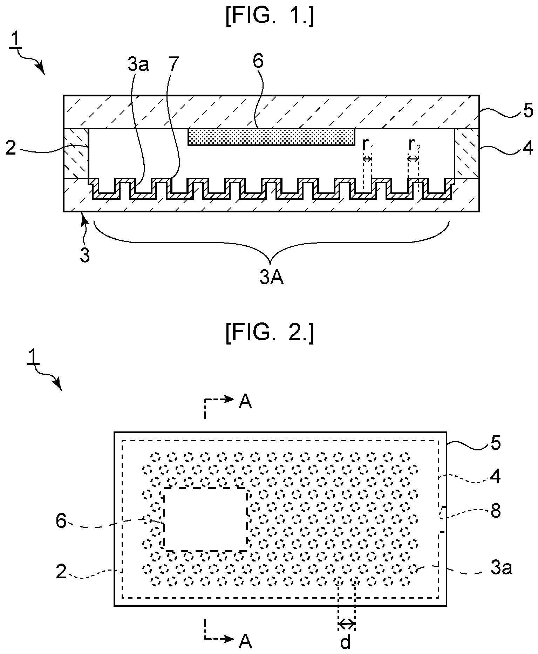

Fig. 1 is a front sectional view of an inspecting instrument according to a first embodiment of the present invention. Fig. 2 is a plan view of the inspecting instrument according to the first embodiment of the present invention. Fig. 1 shows a sectional view of the inspecting instrument along a line A-A in Fig. 2.

An inspecting instrument 1 illustrated in Figs. 1 and 2 includes a compound (first compound) 6 capable of producing a granular substance through a reaction with a test substance, and a wall that has a periodic structure resulting from a plurality of recesses or a plurality of protrusions. The plurality of recesses or the plurality of protrusions include a refractive index adjusting layer 7 on surfaces thereof. In the present embodiment, the wall is a bottom 3 of the inspecting instrument 1. The bottom 3 has a periodic structure 3A (the periodic structure of the wall) on a surface thereof. The refractive index adjusting layer 7 is disposed on a surface of a periodic structure in a wall X. The wall X has, on a surface thereof, the periodic structure resulting from a plurality of recesses or a plurality of protrusions. The refractive index adjusting layer 7 is disposed on the entire surfaces of the plurality of recesses or the plurality of protrusions. Therefore, the refractive index adjusting layer 7 also has a periodic structure. In place of the compound 6 capable of producing a granular substance through a reaction with a test substance, a compound (second compound) that is capable of being bound with a test substance and is a granular substance may be used.

The inspecting instrument 1 includes a side wall 4 provided upright from the bottom 3 on a side end of the periodic structure 3A in the bottom 3. The inspecting instrument 1 includes a storage portion 2 surrounded by the bottom 3, the side wall 4, and the refractive index adjusting layer 7. The storage portion 2 is capable of storing a test substance-containing solution.

The inspecting instrument 1 includes a lid 5 disposed on an opposite end to a bottom-3-end of the side wall 4. The compound 6 is disposed on a surface of the lid 5. When the test substance-containing solution is stored in the storage portion 2, the disposition of the lid 5 on the storage portion 2 enables the solution to be brought into contact with the compound 6 in the storage portion 2. Therefore, the test substance can be easily reacted with the compound 6. In the present embodiment, the compound 6 is disposed above the periodic structure of the wall.

The test substance-containing solution before the reaction or the binding of the test substance with the compound 6 is, for example, the test substance-containing solution itself having been introduced into the inspecting instrument. The test substance-containing solution after the reaction or the binding of the test substance with the compound 6 is, for example, a reaction solution containing a granular substance produced through the reaction or the binding of the test substance with the compound 6.

The inspecting instrument 1 is a microchip that includes the bottom 3 including the refractive index adjusting layer 7, the side wall 4, and the lid 5. As illustrated in Fig. 2, the inspecting instrument 1 includes an introduction portion 8. The introduction portion 8 is a portion where the side wall 4 is not provided. The test substance-containing solution can be introduced through the introduction portion 8 into the storage portion 2. The introduction portion may be provided on the lid. The inspecting instrument may include a fine flow path connected to the introduction portion 8 or may include, for example, a space for pre-treating the test substance or a waste liquid path.

The concentration of the test substance can be, as described below, measured by irradiating the periodic structure of the wall with inspecting light while the test substance-containing solution is stored in the inspecting instrument (storage portion), and measuring the intensity of light reflected from the periodic structure of the wall. From the viewpoint of transmitting the inspecting light, the lid is preferably transparent. A material for the bottom, the side wall, and the lid of the inspecting instrument is not particularly limited and is, for example, a resin or glass.

The bottom 3 and the periodic structure 3A (the periodic structure of the wall) include a plurality of recesses 3a. In the present embodiment, the shape of the recesses 3a is a column shape.

The recesses 3a are regularly disposed. In the present embodiment, the recesses 3a are disposed in plural rows at equal intervals in a first direction (vertical direction in Fig. 2) and a second direction (horizontal direction in Fig. 2) perpendicular to the first direction. The recesses 3a have a two-dimensional periodic structure. The periodic structure of the wall resulting from the plurality of recesses 3a has a hexagonal lattice structure. The periodic structure of the wall X also has a hexagonal lattice structure.

A radius $r_1$ of a recess in the periodic structure of the wall is the radius of the upper surface of the recess. A radius $r_2$ of a protrusion in the periodic structure of the wall is the radius of the upper surface of the protrusion. Further, a period d of the periodic structure of the wall is the center-to-center distance between recesses most adjacent to each other.

In the periodic structure of the wall and the periodic structure of the wall X, the plurality of recesses may be regularly disposed or the plurality of protrusions may be regularly disposed.

The refractive index adjusting layer need not be disposed on the entire surface of the periodic structure of the wall X. The refractive index adjusting layer may be disposed only on upper surfaces of the recesses or the protrusions or only on side surfaces of the recesses or the protrusions in the periodic structure of the wall X. Accordingly, the refractive index adjusting layer need not be disposed on the entire surfaces of the plurality of recesses or the plurality of protrusions. The refractive index adjusting layer may be disposed only on upper surfaces of the recesses or the protrusions or only on side surfaces of the recesses or the protrusions in the plurality of recesses or the plurality of protrusions. The phrase "upper surfaces of the recesses or the protrusions in the periodic structure of the wall X" means bottom surfaces of the recesses when the periodic structure of the wall X is formed of the recesses, and means tip surfaces of the protrusions when the periodic structure of the wall X is formed of the protrusions. From the viewpoint of allowing the present invention to effectively exhibit its effects, the refractive index adjusting layer is preferably disposed over the entire surface of the periodic structure of the wall X.

The shape and the disposition of the protrusions or the recesses can be appropriately changed according to the direction and the angle of the irradiation with the inspecting light. The tip shape of the protrusions or the recesses may be a flat or a curved shape or may be a dot shape. The protrusions or the recesses may be a groove shape. For example, the shape of the protrusions or the recesses may be a rectangular column shape, a pyramid shape, or a circular cone shape. The periodic structure of the wall and the periodic structure of the wall X may each be a one-dimensional periodic structure.

The period of the periodic structure of the wall and the period of the periodic structure of the wall X are each preferably the particle size or more of the granular substance.

The opening area between protrusions or the opening area of a recess in the wall X is preferably 2500 nm$^2$ or more, more preferably 10000 nm$^2$ or more, and preferably 640000 nm$^2$ or less, more preferably 160000 nm$^2$ or less. The opening area between the protrusions and the opening area of the recess in the wall X are the opening areas obtained by removing a thickness portion of the refractive index adjusting layer from the opening area between the protrusions and the opening area of the recess in the wall, respectively, and are the opening area of the protrusion or the recess in the periodic structure of the wall X.

The height of the protrusions or the depth of the recesses in the wall X is preferably 50 nm or more, more preferably 100 nm or more, and preferably 800 nm or less, more preferably 400 nm or less. The height of the protrusions and the depth of the recesses in the wall X are the height or the depth obtained by removing the thickness of the refractive index adjusting layer from the height of the protrusions and the depth of the recesses in the wall, respectively, and are the height of the protrusions or the depth of the recesses in the periodic structure of the wall X.

The opening area between the protrusions, the opening area of the recess, the height of the protrusions, and the depth of the recesses that are described above and are in the wall X can be appropriately changed according to, for example, the type of the granular substance produced through the reaction of the test substance with the compound, and the shape of the protrusions or the recesses. The adjustment of the period of the periodic structure and the shape and the size of the protrusions or the recesses enables the intensity of the reflected light to be optimized.

There are sometimes difficulty in which a plurality of opening areas and the heights of the plurality of protrusions or the depths of the plurality of recesses, respectively, perfectly coincide with each other. The period of the periodic structure may be varied within a range not to greatly affect the accuracy of measuring the concentration. From the viewpoint of effectively reducing noise in the measurement, the standard deviation of a plurality of opening areas between the protrusions or a plurality of opening areas of the recesses in the periodic structure is preferably 10% or less, more preferably 5% or less.

Examples of the test substance include a glycan, glycolipid, a protein, a peptide, and DNA. Examples of the glycan include Mac-2 binding protein glycan. Examples of the glycolipid include endotoxin, peptide glucan, and β-D-glucan. Examples of the protein include a tumor marker, urine protein, and an amyloid. Examples of the tumor marker include PSA as a prostate cancer marker, CEA as a colorectal cancer marker, CA15-3 as a breast cancer marker, and AEP as a lung cancer marker. Examples of the peptide include brain natriuretic peptide (BNP), atrial natriuretic peptide (ANP), and amyloid β and tau protein as Alzheimer's disease markers.

Examples of the compound (the first compound or the second compound) include an enzyme, an enzyme-containing reagent, latex particles, gold particles, silver particles, a support with an antibody, and a support with complementary DNA. Examples of the enzyme-containing reagent include LAL reagent. Examples of the support with an antibody include latex with an antibody and metal nanoparticles with an antibody, such as gold particles with an antibody and silver particles with an antibody.

For example, when the test substance is glycolipid, the compound capable of producing a granular substance through a reaction with the glycolipid may be an enzyme capable of being reacted with the glycolipid.

In the inspecting instrument 1, the test substance is endotoxin and the compound 6 is LAL reagent. The compound 6 produces a gel granular substance through a reaction with the test substance. In the inspecting instrument 1, the period d (distance between recesses 3a) of the periodic structure of the wall is 460 nm. The deposition of the granular substance on the recesses 3a and the refractive index adjusting layer 7 enables the intensity of the light reflected from the periodic structure 3A of the wall to be greatly changed. Specifically, the inspecting instrument 1 is capable of drastically lowering the intensity of the light reflected from the periodic structure 3A of the wall. Accordingly, the concentration of a low-concentration test substance can be measured with high accuracy.

Examples of the liquid include water, an organic liquid, and an ionic liquid. Generally, water is suitably used.

The size of the recesses is the opening area at the opening end of each of the recesses (the opening area of the recess). The size between protrusions is the opening area at the opening end between the protrusions (the opening area between the protrusions). The size of one recess and the size of one space between protrusions may be smaller than the size of the granular substance. In this case, the granular substance covers the periodic structure of the wall. The intensity of the light reflected from the bottom of the inspecting instrument can be greatly changed.

The test substance may contain an antigen or may be an antigen such as a marker. In this case, the compound (the first compound or the second compound) preferably contains an antibody. The compound may be, for example, latex with an antibody. The antibody is capable of being bound with the antigen. The binding of the test substance with the compound enables a granular substance in aggregation to be produced. Also when the granular substance in aggregation covers the periodic structure of the wall, the intensity of the light reflected from the periodic structure of the wall can be greatly changed.

The periodic structure 3A of the wall and the refractive index adjusting layer 7 may be provided on a part of the bottom 3. The periodic structure 3A of the wall and the refractive index adjusting layer 7 are preferably provided at least at a position facing the compound 6. At least the periodic structure 3A of the wall is preferably provided at a position facing the compound 6. In this case, the granular substance (granular substance or the like produced through the reaction of the test substance with the compound 6) can be efficiently deposited on the periodic structure 3A of the wall and the refractive index adjusting layer 7 by gravity.

The periodic structure of the wall X can be shaped, for example, at the time of molding the storage portion. Alternatively, the periodic structure of the wall X may be formed by shaping processing of a surface of the storage portion after the storage portion having no periodic structure is obtained.

The wall having the periodic structure on the surface thereof may be the side wall of the inspecting instrument. In this case, the granular substance can be deposited on the periodic structure, for example, by electrophoresis.

The refractive index adjusting layer can be formed, for example, by sputtering as described below.

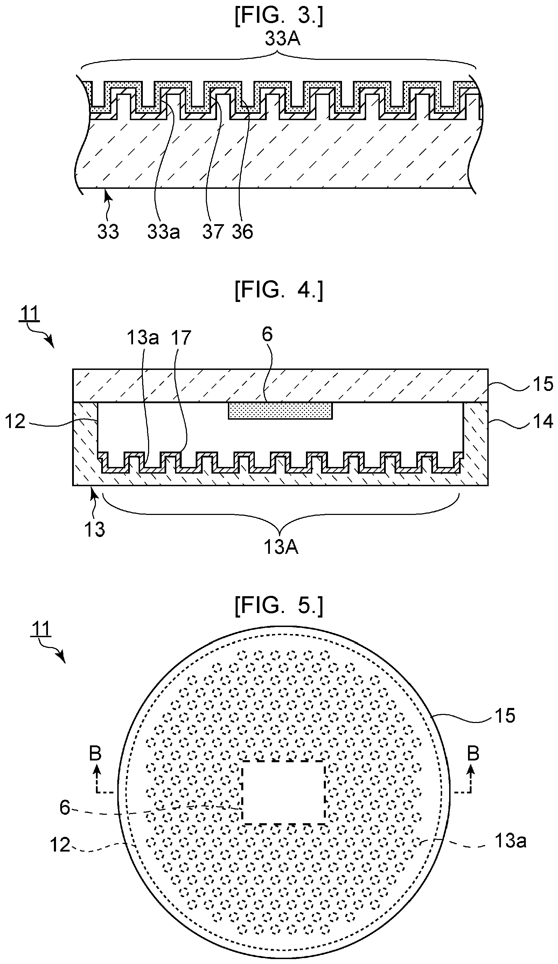

Fig. 3 is an enlarged front sectional view of an inspecting instrument according to a second embodiment of the present invention.

The inspecting instrument of the present embodiment includes a compound (first compound) 36 capable of producing a granular substance through a reaction with a test substance, and a wall that has a periodic structure resulting from a plurality of recesses or a plurality of protrusions. The plurality of recesses or the plurality of protrusions include a refractive index adjusting layer 37 on surfaces thereof. In the present embodiment, the wall is a bottom 33 of the inspecting instrument. The bottom 33 has a periodic structure 33A (the periodic structure of the wall) on a surface thereof. The refractive index adjusting layer 37 is disposed on a surface of a periodic structure in a wall X. The wall X has, on a surface thereof, the periodic structure resulting from a plurality of recesses or a plurality of protrusions. The refractive index adjusting layer 37 is disposed on the entire surfaces of the plurality of recesses or the plurality of protrusions. Therefore, the refractive index adjusting layer 37 also has a periodic structure. In the periodic structure 33A of the wall, the plurality of recesses are regularly disposed. The bottom 33 and the periodic structure 33A include a plurality of recesses 33a. The inspecting instrument of the present embodiment is different from the inspecting instrument 1 in that the compound 36 is disposed on a surface of the refractive index adjusting layer 37. The wall having the periodic structure on the surface thereof may be a side wall of the inspecting instrument. In place of the compound 36 capable of producing a granular substance through a reaction with a test substance, a compound (second compound) that is capable of being bound with a test substance and is a granular substance may be used. In the inspecting instrument of Fig. 3, however, the compound 36 capable of producing a granular substance through a reaction with a test substance is preferably used.

In the present embodiment, the granular substance is produced on surfaces of the refractive index adjusting layer 37 and the periodic structure 33A of the wall. Thus, it is possible to effectively shorten the time from the start of the reaction to the state where the intensity of light reflected from the periodic structure 33A of the wall is greatly changed. When the refractive index adjusting layer is not disposed on the entire surfaces of the plurality of recesses or the plurality of protrusions, the compound may also be disposed on the surface of the periodic structure 33A of the wall, and the granular substance may also be produced on the surface of the periodic structure 33A of the wall and the surface of the refractive index adjusting layer 37.

Fig. 4 is a front sectional view of an inspecting instrument according to a third embodiment of the present invention. Fig. 5 is a plan view of the inspecting instrument according to the third embodiment of the present invention. Fig. 4 shows a sectional view of the inspecting instrument along a line B-B in Fig. 5.

An inspecting instrument 11 includes a petri dish and a lid 15. The petri dish includes a wall on which a periodic structure 13A resulting from a plurality of recesses or a plurality of protrusions is formed. The plurality of recesses or the plurality of protrusions include a refractive index adjusting layer 17 on surfaces thereof. In the present embodiment, the wall is a bottom 13 of the petri dish. The petri dish further includes a side wall 14 provided upright from an outer periphery of the bottom 13. The refractive index adjusting layer 17 is disposed on a surface of a periodic structure in a wall X. The wall X has, on a surface thereof, the periodic structure resulting from a plurality of recesses or a plurality of protrusions. The refractive index adjusting layer 17 is disposed on the entire surfaces of the plurality of recesses or the plurality of protrusions. Therefore, the refractive index adjusting layer 17 also has a periodic structure. In the periodic structure 13A of the wall, the plurality of recesses are regularly disposed. The bottom 13 and the periodic structure 13A include a plurality of recesses 13a.

The inspecting instrument 11 is formed as one instrument (vessel), with the bottom 13 and the side wall 14 integrally formed. The inspecting instrument 11 includes a storage portion 12 surrounded by the bottom 13, the side wall 14, and the refractive index adjusting layer 17.

In the inspecting instrument 11, the reaction of the test substance with the compound 6 can be started by storing the test substance-containing solution in the storage portion 12 and then installing the lid 15 on the storage portion 12.

Meanwhile, the test substance may be introduced into the storage portion 12 after a liquid containing no test substance is stored in the storage portion 12. The irradiation with light for measuring the concentration of the test substance in the liquid may be performed while the lid has been removed. In this case, the lid need not be transparent.

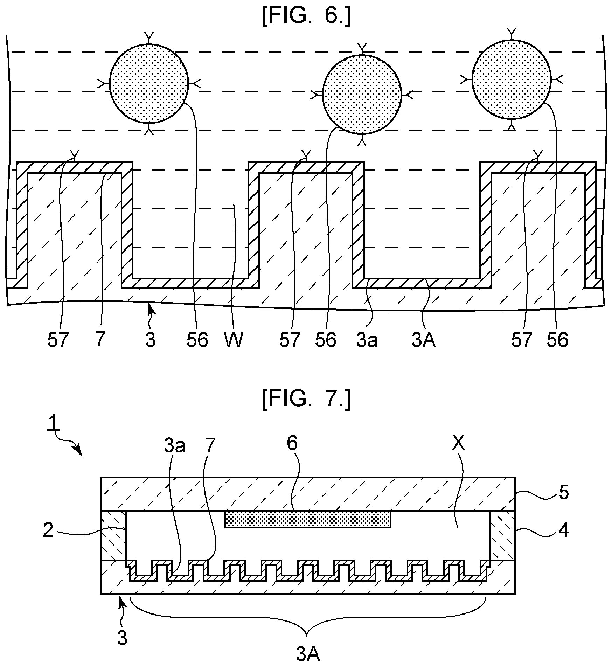

Fig. 6 is an enlarged front sectional view of an inspecting instrument according to a fourth embodiment of the present invention.

The inspecting instrument of the present embodiment includes a compound (second compound) 56 that is capable of being bound with a test substance and is a granular substance, an antibody 57, and a wall that has a periodic structure resulting from a plurality of recesses or a plurality of protrusions. The plurality of recesses or the plurality of protrusions include a refractive index adjusting layer 7 on surfaces thereof. The compound 56 contains an antibody. The compound 56 is, for example, latex with an antibody and is a granular substance. In the present embodiment, as the test substance, an antigen that is bound with the compound 56 is assumed to be used. The antibody 57 is preferably an antibody similar to the antibody contained in the compound 56. In Fig. 6, the antibody is schematically illustrated by a Y-shape.

In Fig. 6, the wall is a bottom 3 of the inspecting instrument. The bottom 3 has a periodic structure 3A (the periodic structure of the wall) on a surface thereof. The refractive index adjusting layer 7 is disposed on a surface of a periodic structure in a wall X. The wall X has, on a surface thereof, the periodic structure resulting from a plurality of recesses or a plurality of protrusions. The refractive index adjusting layer 7 is disposed on the entire surfaces of the plurality of recesses or the plurality of protrusions. Therefore, the refractive index adjusting layer 7 also has a periodic structure. In the periodic structure 3A of the wall, the plurality of recesses are regularly disposed. The bottom 3 and the periodic structure 3A of the wall include a plurality of recesses 3a. The inspecting instrument includes a side wall provided upright from the bottom 3 on a side end of the periodic structure 3A in the bottom 3. The inspecting instrument includes a storage portion surrounded by the bottom 3 and the side wall. The wall having the periodic structure on the surface thereof may be a side wall of the inspecting instrument.

The inspecting instrument of the present embodiment includes a solution W disposed in the storage portion. The solution W contains the compound 56. The antibody 57 is disposed only on tip surfaces of the protrusions in the refractive index adjusting layer 7. The inspecting instrument of the present invention is capable of binding the test substance with the compound 56 during the inspection and is capable of binding the test substance with the antibody 57.

In the present embodiment, the test substance bound with the compound 56 can be selectively deposited on the tip surfaces of the protrusions. In the present embodiment, the refractive index is greatly changed on the tip surfaces of the protrusions, so that the intensity of the light reflected from the refractive index adjusting layer 7 can be greatly changed.

The compound may be disposed similarly to in the first embodiment. The inspecting instrument need not include the antibody 57. Also in this case, the granular substance in which the test substance is bound to the compound is deposited on the periodic structure of the wall, so that the refractive index can be greatly changed.

The antibody 57 may be disposed on bottom surfaces of the recesses in the refractive index adjusting layer 7 or may be disposed on side surfaces of the protrusions or the recesses in the refractive index adjusting layer 7. From the viewpoint of greatly changing the refractive index and measuring the concentration of the test substance with much higher accuracy, the antibody is preferably disposed on the tip surfaces of the protrusions in the refractive index adjusting layer.

In the present embodiment, the periodic structure 3A of the wall has a period about the same as the wavelength of the light to be measured. A medium having a relatively large change of the refractive index allows, in the period about the same as the wavelength of light, no entry of light with a certain wavelength region and reflects the light, but allows transmission of light with a wavelength other than the certain wavelength region. The wavelength region of the light incapable of entering the medium as described above is called a photonic band gap. Appropriately setting the angle or the like of the light to be applied generates the photonic band gap of the periodic structure of the wall. During the inspection, a peak of the reflected light is generated in the wavelength region of the photonic band gap. Since the peak intensity is greatly changed due to the change of the refractive index, the measurement accuracy can be further increased. Further, the disposition of the refractive index adjusting layer enables the measurement accuracy to be significantly increased. Further, the disposition of the refractive index adjusting layer enables the concentration of a low-concentration test substance to be measured.

With reference to Figs. 7 to 9, one example of an inspecting method using the inspecting instrument 1 illustrated in Fig. 1 is described.

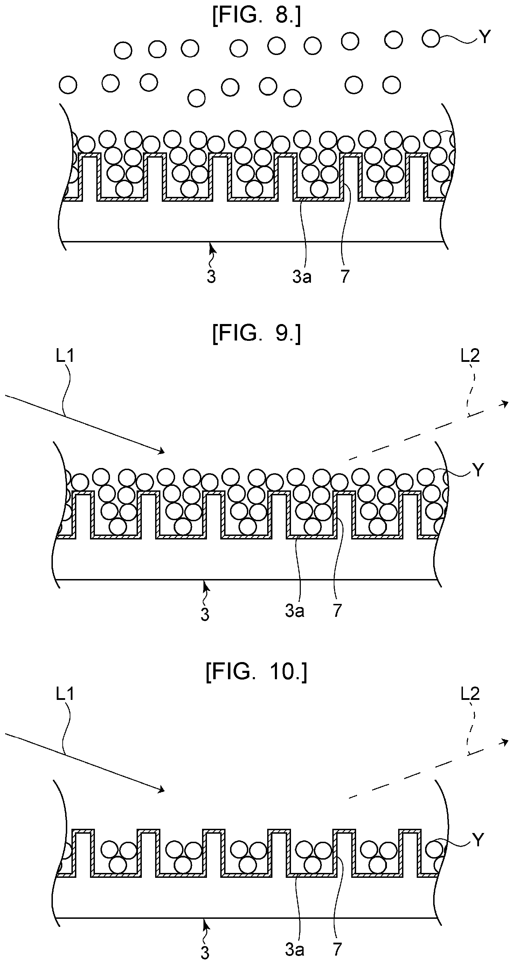

Fig. 7 is a front sectional view for describing the one example of the inspecting method using the inspecting instrument according to the first embodiment of the present invention. Fig. 8 is an enlarged front sectional view for describing the one example of the inspecting method using the inspecting instrument according to the first embodiment of the present invention. Fig. 9 is an enlarged front sectional view for describing the one example of the inspecting method using the inspecting instrument according to the first embodiment of the present invention.

The present inspecting method includes the steps of (1) measuring a reference light intensity, (2) measuring an evaluation light intensity, and (3) acquiring a relative value between the reference light intensity and the evaluation light intensity. The steps (1) to (3) may be performed in this order or may be performed in the order of (2), (1), and (3) .

In the present inspecting method, the steps (1) to (3) are performed on a specimen with a known concentration of a test substance, and a calibration curve is created in advance. For example, a plurality of relationships between (known) concentrations of the test substance and the relative values obtained by the above steps are plotted to create an approximate straight line or an approximate expression. Similarly, the steps (1) to (3) are performed on a specimen with an unknown concentration of the test substance, and the resultant relative value is substituted into the approximate straight line or the approximate expression, so that the concentration of the test substance in the unknown specimen can be determined.

Hereinafter, one example of the steps (1) to (3) is described in detail.

(1) In the step of measuring a reference light intensity, the refractive index adjusting layer 7 and the periodic structure 3A of the wall are irradiated with light and the reflected light intensity is measured, while the liquid has been introduced into the inspecting instrument 1. For example, the light is applied while a liquid containing no test substance is stored in the storage portion 2, and the intensity of light reflected from the refractive index adjusting layer 7 and the periodic structure 3A of the wall can be measured. In this case, as the liquid containing no test substance, a fluid or a solvent that is the same as for the specimen containing the test substance can be used.

(2) In the step of measuring an evaluation light intensity, the refractive index adjusting layer 7 and the periodic structure 3A of the wall are irradiated with light and the reflected light intensity is measured, while a test substance-containing solution X has been introduced into the inspecting instrument 1 and the test substance and the liquid are present. Specifically, the test substance-containing solution X is stored in the storage portion 2 and brought into contact with the compound 6 to start a reaction of the test substance with the compound 6. The refractive index adjusting layer 7 and the periodic structure 3A of the wall are irradiated with light and the reflected light intensity is measured, while the resultant granular substance (e.g., a reaction product of the test substance and the compound 6) is deposited on the refractive index adjusting layer 7 and the periodic structure 3A of the wall. The inspecting instrument 1 does not require removal of the liquid before the irradiation with the light. As illustrated in Fig. 8, a granular substance Y is produced through the reaction of the test substance with the compound 6. The granular substance Y settles out by gravity and is deposited on the refractive index adjusting layer 7.

Next, as illustrated in Fig. 9, the periodic structure of the wall on which the granular substance Y has been deposited is irradiated with inspecting light L1. Next, the intensity of light L2 reflected from the refractive index adjusting layer 7 is measured.

The deposition of the granular substance Y on the periodic structure of the wall lowers the intensity of light applied to the periodic structure of the wall and then reflected on the periodic structure of the wall. Accordingly, the accuracy of measuring the concentration of the test substance can be increased.

(3) In the step of acquiring a relative value between the reference light intensity and the evaluation light intensity, a value of the difference between the reference light intensity and the evaluation light intensity is calculated. The relative value may be defined as a value of the ratio between the reference light intensity and the evaluation light intensity. Further, the relative value may be defined as a value obtained by appropriately applying a mathematical operation to the difference or the ratio.

The reflected light intensity may be measured only with a specific wavelength, or the reflected light intensity may be acquired as a spectrum of a plurality of consecutive wavelengths. Any wavelength can be selected as the wavelength of the light to be applied, and the wavelength can be appropriately selected according to the shape and the size of the periodic structure. Visible light can be typically used, and infrared or ultraviolet light can also be used.

In the above description, described is the case of measuring, as the reference light intensity and the evaluation light intensity, the reflected light at the time of irradiation of the periodic structure of the wall with light. As the reference light intensity and the evaluation light intensity, however, transmitted light may be measured. As long as a change in the optical response can be detected from the surface of the periodic structure of the wall, the light to be detected is not necessarily the direct reflected light or transmitted light but may be light to which some kind of optical operation is applied.

Fig. 10 is an enlarged front sectional view for describing one example of the inspecting method using the inspecting instrument according to the first embodiment of the present invention.

As illustrated in Fig. 10, the intensity of the light L2 reflected on the periodic structure of the wall may be measured while the height of the deposition of the granular substance Y in the recesses 3a is lower than the depth of the recesses 3a (the height of the side surfaces of the recesses 3a). Even when the height of the deposition of the granular substance Y in the recesses 3a does not reach the depth of the recesses 3a (the height of the side surfaces of the recesses 3a), the intensity of the light L2 reflected on the periodic structure of the wall is lowered. Accordingly, the concentration of a low-concentration test substance can be measured. Further, the inspection time can be shortened.

From the viewpoint of further increasing the measurement accuracy, the filling rate of a recess in the wall with the granular substance Y at the time of the measurement is preferably 1% or more, more preferably 10% or more, much more preferably 30% or more, further preferably 50% or more, further more preferably 70% or more, particularly preferably 90% or more, most preferably 95% or more. The filling rate is the volume occupied by the granular substance in the volume of a recess of the wall. When the granular substance is deposited between protrusions, the filling rate is the volume occupied by the granular substance in the volume between the protrusions.

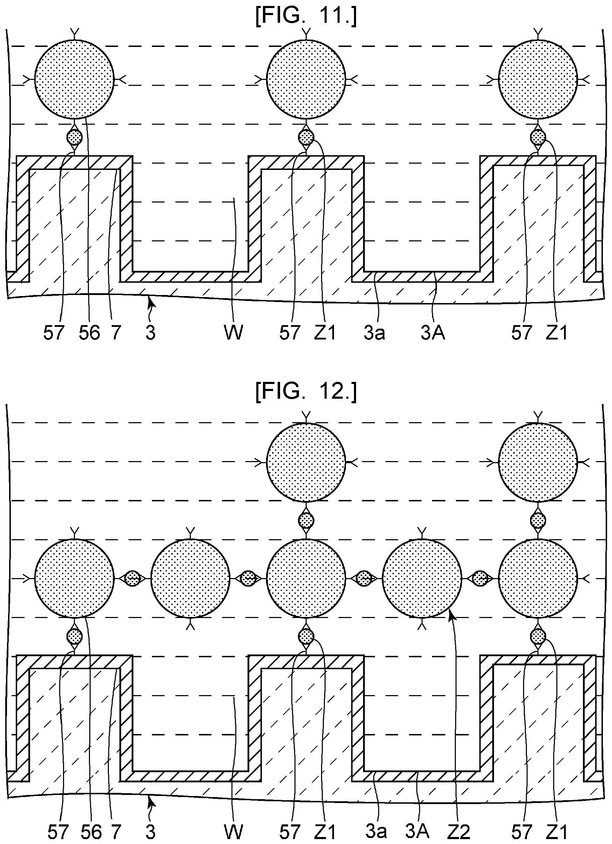

Fig. 11 is an enlarged front sectional view for describing one example of an inspecting method using the inspecting instrument according to the fourth embodiment of the present invention.

As illustrated in Fig. 11, a test substance Z1 in the present inspecting method is an antigen to be bound with the antibody 57 and the antibody contained in the compound 56. The test substance Z1 is bound with the compound 56 contained in the solution W. The compound 56 is a granular substance. The test substance Z1 having been bound with the compound 56 is bound with the antibody 57 disposed on the tip surfaces of the protrusions in the refractive index adjusting layer 7 and selectively deposited on the protrusions. This deposition greatly changes the refractive index of the part to be irradiated with the inspecting light. In the present inspecting method, the inspecting light has a wavelength about the same as the period of the periodic structure 3A of the wall, and a peak of the reflected light is generated in the photonic band gap. The peak intensity of the reflected light is greatly changed due to the change of the refractive index described above. The present inspecting method utilizing the photonic band gap and using the inspecting instrument that includes the refractive index adjusting layer is capable of measuring the concentration of a test substance having a concentration as very low as about 100 pg/ml or less.

Meanwhile, an inspecting instrument can also be used in which the compound is, similarly to in the first embodiment, disposed at a position away from the refractive index adjusting layer and the periodic structure of the wall. In this case, pieces of the test substance Z1 are bound with each other via the compound 56 that is a granular substance, to produce an aggregate Z2 as illustrated in Fig. 12. The granular substance thus aggregated is deposited on the refractive index adjusting layer 7. This case also greatly changes the refractive index of the part to be irradiated with the inspecting light. Also in the case illustrated in Fig. 12, the concentration of a low-concentration test substance can be measured.

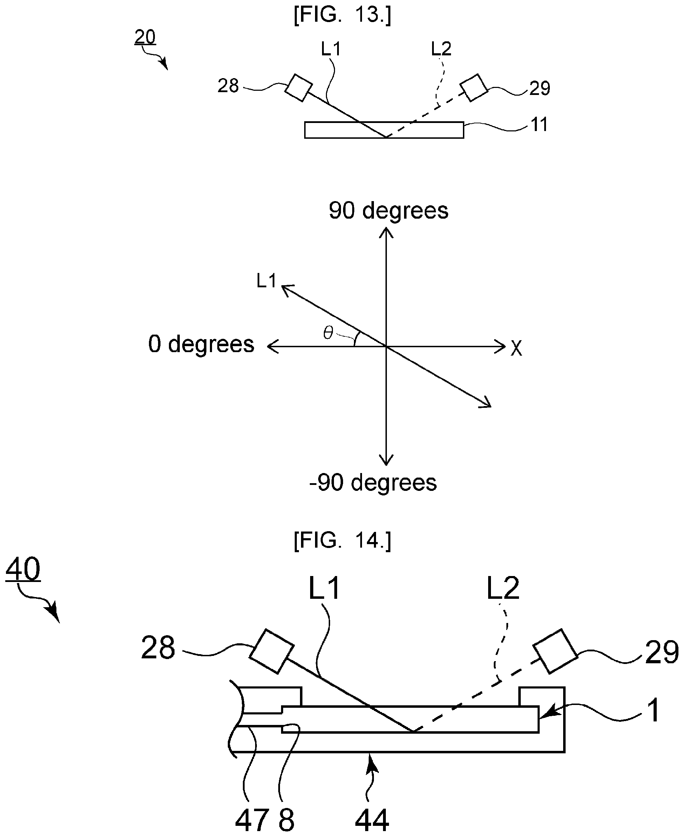

Fig. 13 is a schematic view of an inspecting device according to the first embodiment of the present invention.

An inspecting device 20 includes the inspecting instrument 11 of the third embodiment, a light source 28, and a measuring device 29. The light source 28 emits the light L1 to the periodic structure of the wall including the recesses of the inspecting instrument 11. The intensity of the light L2 reflected on the periodic structure of the wall is measured by the measuring device 29. The inspecting device 20 is capable of measuring the concentration of the test substance with high accuracy by the inspecting method described above.

The inspecting instrument is, as illustrated in Fig. 13, used by irradiating the periodic structure of the wall with light at an incident angle $\theta$ of -90 degrees or more and 90 degrees or less. The incident angle $\theta$ is an incident direction of light with respect to the surface of the wall having the periodic structure and is more specifically an angle formed between a direction X along the surface of the wall having the periodic structure and a direction of the incident light L1. The minimum value of the incident angle $\theta$ is -90 degrees and the maximum value is 90 degrees. The incident angle $\theta$ represented by a negative value means that the back side of the surface of the periodic structure of the wall is irradiated with the light. From the viewpoint of allowing the present invention to further effectively exhibit its effects, the incident angle $\theta$ is preferably -30 degrees or more and 30 degrees or less, more preferably 0 degrees or more and 30 degrees or less, further preferably 0 degrees. The light source 28 and the measuring device 29 may be installed at an identical location.

Fig. 14 is a schematic view of an inspecting device according to the second embodiment of the present invention.

An inspecting device 40 includes the inspecting instrument 1 of the first embodiment, an installation portion 44 on which the inspecting instrument 1 is installed, a light source 28, and a measuring device 29.

The installation portion 44 includes a flow path 47 through which the test substance-containing solution is sent. The flow path 47 is connected to the introduction portion 8 of the inspecting instrument 1. The introduction of the test substance-containing solution through the flow path 47 into the storage portion of the inspecting instrument 1 enables the reaction of the test substance with the compound to be started. The inspecting method described above is capable of measuring the concentration of the test substance with high accuracy. The installation of the inspecting instrument 1 on the installation portion 44 enables an easy inspection.

The inspecting instrument may be a microfluidic device. The microfluidic device may include a compound capable of producing a granular substance or a compound that is capable of being bound with a test substance and is granular substance, and a wall. The compound may be disposed in a microchannel of the microfluidic device. The wall may be an inner wall of the microchannel in the microfluidic device. The inspecting device may be a microfluidic device including the light source and the measuring device.

The inspecting instrument according to the present invention is capable of suitably measuring the concentration of a test substance at, for example, 1 ag/mL or more and 100 pg/mL or less in a liquid. The inspecting instrument according to the present invention is capable of suitably measuring the concentration of a test substance, which is contained in a test substance-containing solution having a refractive index of, for example, 1.3 or more and 1.5 or less, in the liquid.

Hereinafter, the periodic structure of the wall or the wall X, the wall X, and the refractive index adjusting layer are described in further detail.

Periodic structure:

Examples of the periodic structure of the wall and the periodic structure of the wall X include an orthorhombic lattice structure, a rectangular lattice structure, a face-centered lattice structure, a hexagonal lattice structure, and a square lattice structure. From the viewpoint of further easily adjusting the wavelength of the photonic band gap of the periodic structure of the wall and the periodic structure of the wall X and allowing the present invention to further effectively exhibit its effects, the periodic structure of the wall and the periodic structure of the wall X are each preferably an orthorhombic lattice structure, a rectangular lattice structure, a hexagonal lattice structure, or a square lattice structure.

Wall X:

A material for the wall X is, for example, a resin or glass.

Examples of the resin include a polyethylene terephthalate resin, a polyethylene naphthalate resin, a cycloolefin polymer resin, a cycloolefin copolymer resin, a polyimide resin, a polycarbonate resin, a polyurea resin, a polystyrene resin, a polyester resin, and a polymethylmethacrylate resin. The resins may be used singly or in combination of two or more thereof.

Refractive index adjusting layer:

From the viewpoint of measuring the concentration of the test substance in the liquid and the viewpoint of measuring the concentration of the test substance with high accuracy even when the concentration of the test substance is significantly low, the refractive index adjusting layer preferably has a refractive index greater than the refractive index of the test substance-containing solution.

The refractive index adjusting layer has a refractive index of preferably 0.3 or more greater, more preferably 0.4 or more greater, much more preferably 0.5 or more greater, further preferably 0.6 or more greater, particularly preferably 0.7 or more greater, most preferably 0.8 or more greater than the refractive index of the test substance-containing solution. Such a refractive index adjusting layer enables the concentration of the test substance to be well measured in the liquid, and enables the concentration of the test substance to be measured with much higher accuracy even when the concentration of the test substance is significantly low.

The refractive index of the refractive index adjusting layer and the refractive index of the test substance-containing solution can be measured using a spectroscopic ellipsometer (for example, "UVISEL2" manufactured by HORIBA, Ltd.).

For facilitating the adjustment of the refractive index in the above preferable range, the refractive index adjusting layer preferably contains a metallic element or an organic substance. The refractive index adjusting layer may be a compound containing a metallic element and a non-metallic element. Examples of the metallic element include zinc, silver, gold, titanium, aluminum, tin, copper, iron, molybdenum, niobium, titanium, platinum, tungsten, chromium, tin, nickel, tantalum, zirconium, hafnium, yttrium, bismuth, antimony, and indium. Examples of the organic substance include diamond-like carbon (DLC). The metallic elements may be used singly or in combination of two or more thereof. Examples of the compound containing a metallic element and a non-metallic element include silicon carbide (SiC).

The refractive index adjusting layer may be a metal layer or a metal oxide layer. Examples of the metal oxide layer include a $ZnSnO_3$ layer, a zinc oxide layer, a chromium oxide layer, a ferric oxide layer, and a titanium oxide layer.

The refractive index adjusting layer is more preferably a silicon layer. Examples of silicon include monocrystalline silicon, polycrystalline silicon, microcrystalline silicon, and amorphous silicon.

The silicon layer is preferably a monocrystalline silicon layer, a polycrystalline silicon layer, a microcrystalline silicon layer, or an amorphous silicon layer, and is more preferably an amorphous silicon layer. The amorphous silicon may be hydrogenated amorphous silicon.

The formation of the refractive index adjusting layer as a silicon layer enables the refractive index of the refractive index adjusting layer to be further increased, and the formation of the refractive index adjusting layer as an amorphous silicon layer enables the refractive index of the refractive index adjusting layer to be particularly increased. When being a silicon layer such as an amorphous silicon layer, the refractive index adjusting layer can have a refractive index of, for example, 4.5. Therefore, the difference in the refractive index between the refractive index adjusting layer and the test substance-containing solution can be significantly increased to enable the concentration of the test substance to be measured with much higher accuracy.

A method for forming the refractive index adjusting layer is, for example, sputtering (a reactive sputtering method and an RF sputtering method) and a vapor deposition method (a plasma vapor deposition method, etc. and a vacuum vapor deposition method (an EB vapor deposition method, an ion plating method, and an IAD method)). From the viewpoint of well forming the refractive index adjusting layer on the surface of the periodic structure of the wall X, the refractive index adjusting layer is preferably formed by sputtering and is preferably a sputtering film.

The average thickness of the refractive index adjusting layer disposed on the upper surfaces of the recesses or the protrusions in the periodic structure of the wall X is defined as an average thickness (1) of the refractive index adjusting layer. The average thickness of the refractive index adjusting layer disposed on the side surfaces of the recesses or the protrusions in the periodic structure of the wall X is defined as an average thickness (2) of the refractive index adjusting layer.

From the viewpoint of measuring the concentration of the test substance with much higher accuracy, the refractive index adjusting layer has an average thickness (1) of preferably 1 nm or more, more preferably 10 nm or more, further preferably 40 nm or more, and preferably 240 nm or less, more preferably 200 nm or less, further preferably 150 nm or less, particularly preferably 100 nm or less.

The refractive index adjusting layer has a ratio (the average thickness (1) of the refractive index adjusting layer/the average thickness (2) of the refractive index adjusting layer) of the average thickness (1) to the average thickness (2) of preferably 1.0 or more, more preferably 2 or more, and preferably 10 or less, more preferably 4 or less. The refractive index adjusting layer having a ratio (the average thickness (1) of the refractive index adjusting layer/the average thickness (2) of the refractive index adjusting layer) of the above lower limit or more and the above upper limit or less enables the concentration of the test substance to be measured with much higher accuracy.

The average thickness (1) of the refractive index adjusting layer and the average thickness (2) of the refractive index adjusting layer can be measured by observing a section of the refractive index adjusting layer using, for example, an FE-TEM (e.g., "JEM-ARM200F" manufactured by JEOL Ltd.). Any five or more points are selected on a sectional TEM image obtained by the FE-TEM, with the points separated from each other at a distance of 100 nm or more, and the average value of the thicknesses measured at the points is defined as the average thickness.

In the inspecting method according to the present invention and the inspecting instrument according to the present invention, the test substance-containing solution may be a solution containing a biological sample. Examples of the biological sample include blood, spinal fluid, urine, feces, a tissue, a cell, a nucleic acid extract, and a protein extract. Particularly, when the biological sample is blood, the refractive index adjusting layer is preferably a silicon layer, more preferably an amorphous silicon layer. The blood may contain, for example, a known drug such as an anticoagulant (e.g., citric acid, heparin, or ethylenediaminetetraacetic acid (EDTA)).

Generally, when the concentration of a test substance contained in blood is measured in the blood, the measurement accuracy is sometimes lowered due to an influence of hemoglobin leaked from red blood cells. For example, the measurement accuracy is sometimes lowered due to a specific absorption spectrum of hemoglobin. Amorphous silicon, however, effectively absorbs light with a wavelength of 650 nm or less and is therefore capable of increasing the measurement accuracy even in the presence of hemoglobin. Further, the amorphous silicon layer can have a refractive index of up to about 4.5, to enable the concentration of the test substance contained in the blood to be measured with much higher accuracy.

Hereinafter, the present invention is described in further detail with reference to specific examples.

(Example 1)

Using the inspecting instrument of the fourth embodiment, a relationship between the concentration of cystatin C and the wavelength shift between reflection spectra was obtained.

The inspecting instrument of the fourth embodiment was prepared. As the material for the wall X, a polyethylene terephthalate resin having a refractive index of 1.5 was used. The shape of the protrusions in the periodic structure of the wall X was made into a cylindrical shape. A $ZnSnO_3$ layer (refractive index adjusting layer) having a refractive index of 2.0 was formed on the entire surface of the periodic structure of the wall X by sputtering. Thus, the wall (bottom) was formed by the wall X and the refractive index adjusting layer, and the periodic structure (the periodic structure of the wall) is formed on the surface of the wall. Cystatin C antibody was fixed onto the surface of the refractive index adjusting layer using glutaraldehyde as a fixing solution, and then unreacted aldehyde groups were blocked with ethanolamine. Next, the inspecting instrument was dried. Thus, the surface of the refractive index adjusting layer was modified with the cystatin C antibody. The inspecting instrument used is described below in detail.

Periodic structure of wall: square lattice structure
Period of periodic structure of wall: 460 nm
Radius of protrusions in periodic structure of wall: 115 nm
Wavelength of photonic band gap of periodic structure of wall: 580 nm
Average thickness of refractive index adjusting layer disposed on upper surfaces of protrusions in periodic structure of wall X: 40 nm
Average thickness of refractive index adjusting layer disposed on side surfaces of protrusions in periodic structure of wall X: 20 nm

Next, a solution containing cystatin C and saline was prepared and stored in the storage portion of the inspecting instrument. The solution containing the cystatin C and the saline was prepared at eight concentrations of the cystatin C, i.e., 1 fg/ml, 5 fg/ml, 10 fg/ml, 50 fg/ml, 100 fg/ml, 500 fg/ml, 1 pg/ml, and 5 pg/ml. Further, saline (containing no cystatin C) was also separately stored in the storage portion of the inspecting instrument. The solution containing the cystatin C and the saline, and the saline each had a refractive index of 1.3.

Next, an aqueous solution of latex modified with cystatin C antibody (manufactured by SEKISUI MEDICAL CO., LTD.) was disposed on the lid (cover glass). Next, the aqueous solution of latex modified with cystatin C antibody was stored in the storage portion of the inspecting instrument.

Next, after the storage of the aqueous solution of latex modified with cystatin C antibody, the periodic structure in the inspecting instrument was irradiated with light without removing water, to measure the concentration of the test substance. The measurement was performed for a reflection spectrum A of the reflected light immediately after (0 minutes) the addition of the solution containing the cystatin C and the saline or the addition of the saline and performed for a reflection spectrum B of the reflected light after leaving the solution or the saline to stand for 20 minutes. The surface of the wall having the periodic structure in the inspecting instrument was irradiated with light (white light) at an incident angle of 15 degrees, and the intensity of light reflected (evaluation light intensity) was measured.

The wavelength shift between the reflection spectra of the solution containing the cystatin C and the saline was obtained. The wavelength shift is the difference in peak wavelength between immediately after (0 minutes) the addition of the solution and after the solution was left to stand for 10 minutes. Similarly, the wavelength shift when the saline was stored in the storage portion was obtained.

(Example 2)

A relationship between the concentration of cystatin C and the wavelength shift between reflection spectra was obtained similarly to in Example 1 except that an amorphous silicon layer having a refractive index of 4.5 was formed as the refractive index adjusting layer. The inspecting instrument used is described below in detail.

Periodic structure of wall: square lattice structure
Period of periodic structure of wall: 460 nm
Radius of protrusions in periodic structure of wall: 115 nm
Wavelength of photonic band gap of periodic structure of wall: 680 nm and 820 nm
Average thickness of refractive index adjusting layer disposed on upper surfaces of protrusions in periodic structure of wall X: 40 nm
Average thickness of refractive index adjusting layer disposed on side surfaces of protrusions in periodic structure of wall X: 20 nm

(Comparative Example 1)

The refractive index adjusting layer was not provided. A solution containing cystatin C and saline was prepared at thirteen concentrations of the cystatin C, i.e., 1 fg/ml, 5 fg/ml, 10 fg/ml, 50 fg/ml, 100 fg/ml, 500 fg/ml, 1 pg/ml, 5 pg/ml, 10 pg/ml, 50 pg/ml, 100 pg/ml, 500 pg/ml, and 1 ng/mL. Except for these changes, the relationship between the concentration of cystatin C and the wavelength shift between reflection spectra was obtained similarly to in

Example 1.

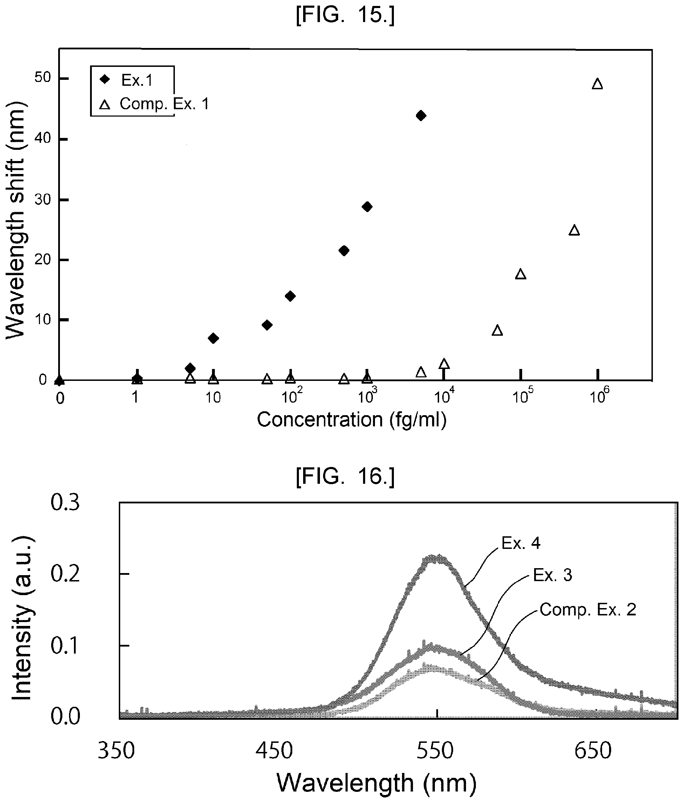

Fig. 15 is a graph illustrating the relationship between the concentration of cystatin C and the wavelength shift between the reflection spectra in Example 1 and Comparative Example 1. In Fig. 15, the horizontal axis (concentration of cystatin C) is represented logarithmically. Fig. 15 also shows the measurement results of the saline (containing no cystatin C) in Example 1 and Comparative Example 1.

As shown in Fig. 15, it is clarified that in Example 1, when the concentration of cystatin C is 1 fg/ml or more, the wavelength shift increases along with an increase in the concentration of cystatin C. In contrast, it is clarified that in Comparative Example 1, when the concentration of cystatin C is 5 pg/ml or more, the wavelength shift increases along with an increase in the concentration of cystatin C. Further, in Example 2 in which the refractive index adjusting layer was an amorphous silicon layer, the wavelength shift was increased compared to Example 1 in which the refractive index adjusting layer was a $ZnSnO_3$ layer. Accordingly, it is clarified that the present invention is capable of performing a high-accuracy inspection on the test substance at a concentration of around 1 fg/ml and enables an inspection for the test substance also in a very low concentration range.

(Example 3)

Using the inspecting instrument of the third embodiment, a relationship among the average thickness of the refractive index adjusting layer, the spectrum wavelength, and the reflected light intensity was obtained.

The inspecting instrument of the third embodiment was prepared. As the material for the wall X, a cycloolefin resin having a refractive index of 1.5 was used. The shape of the recesses in the periodic structure of the wall X was made into a cylindrical shape. A $ZnSnO_3$ layer (refractive index adjusting layer) having a refractive index of 2.0 was formed on the entire surface of the periodic structure by sputtering. Thus, the wall (bottom) was formed by the wall X and the refractive index adjusting layer, and the periodic structure (the periodic structure of the wall) is formed on the surface of the wall. The inspecting instrument used is described below in detail.

Periodic structure of wall: square lattice structure
Period of periodic structure of wall: 460 nm
Radius of recesses in periodic structure of wall: 115 nm
Plane area (opening area) of recess in periodic structure of wall: $41.5 \times 10^3$ nm$^2$
Depth of recesses in periodic structure of wall: 200 nm
Wavelength of photonic band gap of periodic structure of wall: 560 nm
Average thickness of refractive index adjusting layer disposed on upper surfaces of recesses in periodic structure of wall X: 20 nm
Average thickness of refractive index adjusting layer disposed on side surfaces of recesses in periodic structure of wall X: 10 nm

Next, the bottom of the inspecting instrument was irradiated with light, and the intensity of light reflected on the bottom was measured.

(Example 4)

The bottom of the inspecting instrument was irradiated with light and the intensity of light reflected on the bottom was measured similarly to in Example 3 except that the average thickness of the refractive index adjusting layer was changed. The inspecting instrument used is described below in detail.

Periodic structure of wall: square lattice structure
Period of periodic structure of wall: 460 nm
Radius of recesses in periodic structure of wall: 115 nm
Plane area (opening area) of recess in periodic structure of wall: $41.5 \times 10^3$ nm$^2$
Depth of recesses in periodic structure of wall: 200 nm
Wavelength of photonic band gap of periodic structure of wall: 640 nm and 800 nm
Average thickness of refractive index adjusting layer disposed on upper surfaces of recesses in periodic structure of wall X: 40 nm
Average thickness of refractive index adjusting layer disposed on side surfaces of recesses in periodic structure of wall X: 20 nm

(Comparative Example 2)

The bottom of the inspecting instrument was irradiated with light and the intensity of light reflected on the bottom was measured similarly to in Example 3 except that no refractive index adjusting layer was provided.

Fig. 16 is a graph illustrating the relationship among the average thickness of the refractive index adjusting layer, the spectrum wavelength, and the reflected light intensity in Examples 3 and 4 and Comparative Example 2.

As shown in Fig. 16, it is clarified that the inspecting instrument including the refractive index adjusting layer has a greater intensity of the light reflected on the bottom than the intensity of the light in the inspecting instrument including no refractive index adjusting layer. This result clarifies that the inspecting instrument including the refractive index adjusting layer is capable of measuring the concentration of the test substance with higher accuracy than the inspecting instrument including no refractive index adjusting layer. Further, it is clarified that the intensity of the light reflected on the bottom is greater when the refractive index adjusting layer disposed on the upper surfaces of the recesses in the periodic structure of the wall X has an average thickness of 40 nm than when the refractive index adjusting layer disposed on the upper surfaces of the recesses in the periodic structure of the wall X has an average thickness of 20 nm. This result clarifies that the concentration of the test substance can be measured with higher accuracy when the refractive index adjusting layer disposed on the upper surfaces of the recesses in the periodic structure of the wall X has an average thickness of 40 nm than when the refractive index adjusting layer disposed on the upper surfaces of the recesses in the periodic structure of the wall X has an average thickness of 20 nm.

(Example 5)

The inspecting instrument of the fourth embodiment was prepared. As the material for the wall X, a cycloolefin resin having a refractive index of 1.5 was used. The shape of the protrusions in the periodic structure of the wall X was made into a cylindrical shape. An amorphous silicon layer (refractive index adjusting layer) having a refractive index of 4.5 was formed on the entire surface of the periodic structure of the wall X by sputtering. Thus, the wall (bottom) was formed by the wall X and the refractive index adjusting layer, and the periodic structure (the periodic structure of the wall) is formed on the surface of the wall. Cystatin C antibody was fixed onto the surface of the refractive index adjusting layer using glutaraldehyde as a fixing solution, and then unreacted aldehyde groups were blocked with ethanolamine. Next, the inspecting instrument was dried. Thus, the surface of the refractive index adjusting layer was modified with the cystatin C antibody. Except for the matter described above, a relationship between the concentration of cystatin C and the wavelength shift between reflection spectra was obtained similarly to in Example 1. The inspecting instrument used is described below in detail.

Periodic structure of wall: square lattice structure
Period of periodic structure of wall: 460 nm
Radius of protrusions in periodic structure of wall: 115 nm
Wavelength of photonic band gap of periodic structure of wall: 620 nm and 780 nm
Average thickness of refractive index adjusting layer disposed on upper surfaces of protrusions in periodic structure of wall X: 10 nm
Average thickness of refractive index adjusting layer disposed on side surfaces of protrusions in periodic structure of wall X: 5 nm

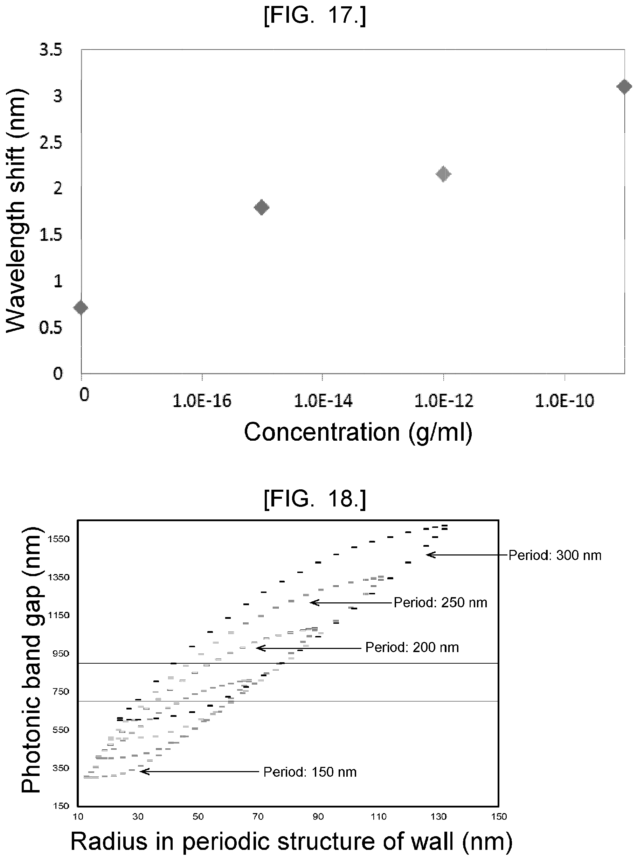

Fig. 17 is a graph illustrating the relationship between the concentration of cystatin C and the wavelength shift between the reflection spectra in Example 5. In Fig. 17, the horizontal axis (concentration of cystatin C) is represented logarithmically. Fig. 17 also shows the measurement result of the saline (containing no cystatin C) in Example 5. In Fig. 17, "1.0E-10" means "$1.0 \times 10^{-10}$," "1.0E-12" means "$1.0 \times 10^{-12}$," "1.0E-14" means "$1.0 \times 10^{-14}$," and "1.0E-16" means "$1.0 \times 10^{-16}$."

(Example 6)

A relationship among the wavelength of a photonic band gap, the period of a periodic structure of a wall, and the radius of protrusions or recesses in the periodic structure of the wall was obtained by simulations. In these simulations, the following dimensions and the like are set.
Periodic structure of wall: square lattice structure
The period was changed to 150 nm, 200 nm, 250 nm, or 300 nm so that the ratio (period/radius) of the period (nm) of the periodic structure of the wall to the radius (nm) of the protrusions or the recesses in the periodic structure of the wall became 0.18.

Refractive index adjusting layer: silicon layer
Average thickness of refractive index adjusting layer disposed on upper surfaces of protrusions in periodic structure of wall X: 10 nm
Average thickness of refractive index adjusting layer disposed on side surfaces of protrusions in periodic structure of wall X: 10 nm

Fig. 18 is a graph illustrating the relationship, which was obtained by the simulations, between the radius of protrusions or recesses in a periodic structure of a wall and the wavelength of a photonic band gap of the periodic structure of the wall. In Fig. 18, the horizontal axis is the radius (nm) of protrusions or recesses in a periodic structure of a wall, and the vertical axis is the wavelength (nm) of a photonic band gap of the periodic structure of the wall.

(Example 7)

The wavelength spectra of photonic band gaps in an inspecting instrument having the following dimensions and the like were obtained by simulations. The resultant wavelength spectra were plotted, with the horizontal axis representing the refractive index of photonic crystals (the refractive index of periodic structures) and the vertical axis representing the maximum wavelength at a wavelength of around 560 nm or the maximum wavelength at a wavelength of around 720 nm, and an inclination (A) of the resultant approximate straight line was obtained.

Refractive index of photonic crystal: 1, 1.05, 1.1, 1.15, 1.2, 1.25, 1.3, and 1.35
Periodic structure of wall: square lattice structure
Period of periodic structure of wall: 300 nm
Radius of protrusions in periodic structure of wall: 54 nm
Refractive index adjusting layer: silicon layer
Average thickness of refractive index adjusting layer disposed on upper surfaces of protrusions in periodic structure of wall X: 10 nm
Average thickness of refractive index adjusting layer disposed on side surfaces of protrusions in periodic structure of wall X: 10 nm

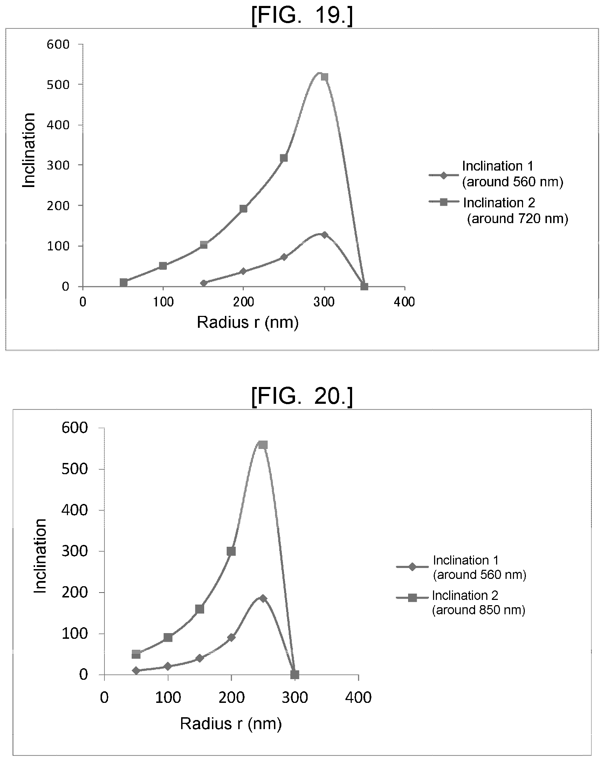

The radius of the protrusions in the periodic structures of the walls was appropriately changed, and an inclination (A) of the approximate straight line was obtained similarly to the above.
A plurality of approximate straight lines were plotted with the horizontal axis representing the radius of the protrusions in the periodic structures of the walls and the vertical axis representing the inclination (A) of the approximate straight lines. Fig. 19 is a graph illustrating a relationship, which was obtained by the simulations, between the radius of the protrusions in the periodic structures of the walls and the inclination (A) of the plurality of approximate straight lines. The approximate straight line having a greater inclination means more excellent detection sensitivity.

(Example 8)

The wavelength spectra of photonic band gaps in an inspecting instrument having the following dimensions and the like were obtained by simulations similarly to in Example 7. The resultant wavelength spectra were plotted, with the horizontal axis representing the refractive index of photonic crystals (the refractive index of periodic structures) and the vertical axis representing the maximum wavelength at a wavelength of around 560 nm or the maximum wavelength at a wavelength of around 850 nm, and an inclination (A) of the resultant approximate straight line was obtained.

Refractive index of photonic crystal: 1, 1.05, 1.1, 1.15, 1.2, 1.25, 1.3, and 1.35
Periodic structure of wall: triangular lattice structure
Period of periodic structure of wall: 460 nm
Radius of protrusions in periodic structure of wall: 125 nm
Refractive index adjusting layer: silicon layer
Average thickness of refractive index adjusting layer disposed on upper surfaces of protrusions in periodic structure of wall X: 10 nm
Average thickness of refractive index adjusting layer disposed on side surfaces of protrusions in periodic structure of wall X: 10 nm

The radius of the protrusions in the periodic structures of the walls was appropriately changed, and an inclination (A) of the approximate straight line was obtained similarly to the above.
A plurality of approximate straight lines were plotted with the horizontal axis representing the radius of the protrusions in the periodic structures of the walls and the vertical axis representing the inclination (A) of the approximate straight lines. Fig. 20 is a graph illustrating a relationship, which was obtained by the simulations, between the radius of the protrusions in the periodic structures of the walls and the inclination (A) of the plurality of approximate straight lines.

**EXPLANATION OF SYMBOLS**

1: Inspecting instrument
2: Storage portion
3: Bottom (wall)
3A: Periodic structure (periodic structure of wall)
3a: Recess (recess of wall)
4: Side wall
5: Lid
6: Compound
7: Refractive index adjusting layer
8: Introduction portion
11: Inspecting instrument
12: Storage portion
13: Bottom (wall)
13A: Periodic structure (periodic structure of wall)
13a: Recess (recess of wall)
14: Side wall
15: Lid
17: Refractive index adjusting layer
20: Inspecting device
28: Light source
29: Measuring device
33: Bottom (wall)
33A: Periodic structure (periodic structure of wall)
33a: Recess (recess of wall)
36: Compound
37: Refractive index adjusting layer
40: Inspecting device
44: Installation portion
47: Flow path
56: Compound
57: Antibody
d: Period
$r_1$, $r_2$: Radius

## Claims

1. An inspecting method for measuring, using a test substance-containing solution containing a test substance and a liquid, a concentration of the test substance, which is contained in the test substance-containing solution, in the liquid,

   the inspecting method using an inspecting instrument (1, 11),
   the inspecting instrument (1, 11) including a wall (3, 13, 33) that has a periodic structure (3A, 13A, 33A) resulting from a plurality of recesses (3a, 13a, 33a) or a plurality of protrusions,
   the plurality of recesses (3a, 13a, 33a) or the plurality of protrusions including a refractive index adjusting layer (7, 17, 37) on surfaces thereof,
   the refractive index adjusting layer (7, 17, 37) being a silicon layer,
   the inspecting method comprising the steps of:

   measuring a reference light intensity by irradiating the periodic structure with light while the liquid has been introduced into the inspecting instrument;
   measuring an evaluation light intensity by irradiating the periodic structure with light while the test substance-containing solution has been introduced into the inspecting instrument and the test substance and the liquid are present; and
   acquiring a relative value between the reference light intensity and the evaluation light intensity, and

the inspecting method comparing the relative value obtained when a specimen with a known concentration of the test substance is introduced into the inspecting instrument, to the relative value obtained when a specimen with an unknown concentration of the test substance is introduced into the inspecting instrument, to determine the concentration of the test substance in the specimen with the unknown concentration of the test substance.

2. The inspecting method according to claim 1, wherein

the inspecting instrument (1, 11) includes a compound (6, 36) capable of producing a granular substance through a reaction with the test substance, or a compound (56) that is capable of being bound with the test substance and is a granular substance, and
in the step of measuring the evaluation light intensity, the evaluation light intensity is measured by irradiating the periodic structure (3A, 13A) with light while the test substance-containing solution has been introduced into the inspecting instrument (1, 11) and the granular substance is deposited on the refractive index adjusting layer (37).

3. . The inspecting method according to any one of claims 1 to 2, - wherein the silicon layer is a monocrystalline silicon layer, a polycrystalline silicon layer, a microcrystalline silicon layer, or an amorphous silicon layer.

4. . The inspecting method according to any one of claims 1 to 3 , wherein the silicon layer is the amorphous silicon layer.

5. . The inspecting method according to any one of claims 1 to 4, - wherein the test substance-containing solution is a solution containing a biological sample.

6. An inspecting instrument (1, 11) being used for measuring, using a test substance-containing solution containing a test substance and a liquid, a concentration of the test substance, which is contained in the test substance-containing solution, in the liquid,

the inspecting instrument (1, 11) comprising a wall (3, 13, 33) that has a periodic structure (3A, 13A, 33A) resulting from a plurality of recesses (3a, 13a, 33a) or a plurality of protrusions,
the plurality of recesses (3a, 13a, 33a) or the plurality of protrusions including a refractive index adjusting layer (7, 17, 37) on surfaces thereof, and
the refractive index adjusting layer (7, 17, 37) being a silicon layer.

7. . The inspecting instrument (1, 11) according to claim 6 wherein the silicon layer is a monocrystalline silicon layer, a polycrystalline silicon layer, a microcrystalline silicon layer, or an amorphous silicon layer.

8. . The inspecting instrument (1, 11) according to any one of claims 6 to 7, wherein the silicon layer is the amorphous silicon layer.

9. . The inspecting instrument (1, 11) according to any one of claims 6 to 8, wherein the refractive index adjusting layer (7, 17, 37) has an average thickness of 1 nm or more and 100 nm or less.

10. . The inspecting instrument (1, 11) according to any one of claims 6 to 9, comprising a compound (6, 36) capable of producing a granular substance through a reaction with the test substance, or a compound (56) that is capable of being bound with the test substance and is a granular substance.

11. . The inspecting instrument (1, 11) according to claim 10, wherein the compound (6) is not in contact with the refractive index adjusting layer (7, 17) and is disposed at a position away from the refractive index adjusting layer (7, 17).

12. . The inspecting instrument (1, 11) according to claim 10 or 11, wherein the compound (36) is disposed on a surface of the refractive index adjusting layer (37).

13. . The inspecting instrument (1, 11) according to any one of claims 10 to 12, wherein

when the inspecting instrument (1, 11) comprises the compound capable (6, 36) of producing a granular substance through a reaction with the test substance, the compound (6, 36) capable of producing a granular

23

substance through a reaction with the test substance is a compound (6, 36) capable of producing a granular substance in aggregation through a reaction with the test substance, and
when the inspecting instrument comprises the compound (56) that is capable of being bound with the test substance and is a granular substance, the compound (56) that is capable of being bound with the test substance and is a granular substance is a compound (56) that is bound with the test substance and is thus capable of producing a granular substance in aggregation.

14. . The inspecting instrument (1, 11) according to any one of claims 10 to 13, comprising the compound (56) that is capable of being bound with the test substance and is a granular substance, wherein

the test substance contains an antigen, and
the compound (56) contains an antibody (57).

15. . The inspecting instrument (1, 11) according to any one of claims 10 to 13, comprising the compound (6, 36) capable of producing a granular substance through a reaction with the test substance, wherein

the test substance is glycolipid, and
the compound (6, 36) is an enzyme capable of being reacted with the glycolipid.

16. . The inspecting instrument (1, 11) according to any one of claims 10 to 13, comprising the compound (6, 36) capable of producing a granular substance through a reaction with the test substance, wherein

the test substance is endotoxin, and
the compound (6, 36) is LAL reagent.

17. . The inspecting instrument (1, 11) according to any one of claims 6 to 16, wherein the test substance-containing solution is a solution containing a biological sample.

18. . The inspecting instrument (1, 11) according to any one of claims 6 to 17, being a microchip.

19. . An inspecting device (20, 40) comprising:

the inspecting instrument (1, 11) according to any one of claims 6 to 18;
a light source (28) for irradiating the periodic structure (3A, 13A) in the inspecting instrument (1, 11) with light; and
a measuring device (29) for measuring an intensity of light emitted from the light source (28) and reflected on the periodic structure (3A, 13A).

**Patentansprüche**

1. Prüfverfahren zur Messung einer Konzentration der in der prüfsubstanzhaltigen Lösung enthaltenen Prüfsubstanz in der Flüssigkeit unter Verwendung einer prüfsubstanzhaltigen Lösung, die eine Prüfsubstanz und eine Flüssigkeit enthält,

wobei das Prüfverfahren ein Prüfgerät (1, 11) verwendet,
wobei das Prüfgerät (1, 11) eine Wand (3, 13, 33) aufweist, die eine periodische Struktur (3A, 13A, 33A) aufweist, die sich aus einer Vielzahl von Vertiefungen (3a, 13a, 33a) oder einer Vielzahl von Erhebungen ergibt,
wobei die Vielzahl von Vertiefungen (3a, 13a, 33a) oder die Vielzahl von Erhebungen eine Schicht zur Einstellung des Brechungsindex (7, 17, 37) auf ihren Oberflächen aufweist,
wobei die Schicht zur Einstellung des Brechungsindex (7, 17, 37) eine Siliziumschicht ist,
wobei das Prüfverfahren die folgenden Schritte umfasst:

Messen einer Intensität von Referenzlicht durch Bestrahlen der periodischen Struktur mit Licht, während die Flüssigkeit in das Prüfgerät eingeführt worden ist,
Messen einer Intensität von Auswertungslicht durch Bestrahlen der periodischen Struktur mit Licht, während die prüfsubstanzhaltige Lösung in das Prüfgerät eingeführt worden ist und die Prüfsubstanz und die Flüssigkeit vorhanden sind; und
Erfassen eines relativen Wertes zwischen der Intensität von Referenzlicht und der Intensität von Auswertungslicht, und

wobei das Prüfverfahren den relativen Wert, der erhalten wird, wenn eine Probe mit einer bekannten Konzentration der Prüfsubstanz in das Prüfgerät eingeführt wird, mit dem relativen Wert vergleicht, der erhalten wird, wenn eine Probe mit einer unbekannten Konzentration der Prüfsubstanz in das Prüfgerät eingeführt wird, um die Konzentration der Prüfsubstanz in der Probe mit der unbekannten Konzentration der Prüfsubstanz zu bestimmen.

2. Prüfverfahren nach Anspruch 1, wobei

das Prüfgerät (1, 11) eine Verbindung (6, 36), die in der Lage ist, durch eine Reaktion mit der Prüfsubstanz eine granulare Substanz zu erzeugen, oder eine Verbindung (56), die in der Lage ist, mit der Prüfsubstanz gebunden zu werden und eine granulare Substanz ist, enthält, und

wobei in dem Schritt des Messens der Intensität von Auswertungslicht die Intensität von Auswertungslicht durch Bestrahlen der periodischen Struktur (3A, 13A) mit Licht gemessen wird, während die prüfsubstanzhaltige Lösung in das Prüfgerät (1, 11) eingeführt worden ist und die granulare Substanz auf der Brechungsindex-Einstellschicht (37) abgelagert ist.

3. Prüfverfahren nach einem der Ansprüche 1 bis 2, wobei die Siliziumschicht eine monokristalline Siliziumschicht, eine polykristalline Siliziumschicht, eine mikrokristalline Siliziumschicht oder eine amorphe Siliziumschicht ist.

4. Prüfverfahren nach einem der Ansprüche 1 bis 3, wobei die Siliziumschicht die amorphe Siliziumschicht ist.

5. Prüfverfahren nach einem der Ansprüche 1 bis 4, wobei die prüfsubstanzhaltige Lösung eine Lösung ist, die eine biologische Probe enthält.

6. Prüfgerät (1, 11), mit dem unter Verwendung einer prüfsubstanzhaltigen Lösung, die eine Prüfsubstanz und eine Flüssigkeit enthält, eine Konzentration der in der prüfsubstanzhaltigen Lösung enthaltenen Prüfsubstanz in der Flüssigkeit gemessen wird,

wobei das Prüfgerät (1, 11) eine Wand (3, 13, 33) umfasst, die eine periodische Struktur (3A, 13A, 33A) aufweist, die sich aus einer Vielzahl von Vertiefungen (3a, 13a, 33a) oder einer Vielzahl von Erhebungen ergibt,

wobei die Vielzahl von Vertiefungen (3a, 13a, 33a) oder die Vielzahl von Erhebungen eine Schicht zur Einstellung des Brechungsindex (7, 17, 37) auf ihren Oberflächen aufweist, und

die Schicht zur Einstellung des Brechungsindex (7, 17, 37) eine Siliziumschicht ist.

7. Prüfgerät (1, 11) nach Anspruch 6, wobei die Siliziumschicht eine monokristalline Siliziumschicht, eine polykristalline Siliziumschicht, eine mikrokristalline Siliziumschicht oder eine amorphe Siliziumschicht ist.

8. Prüfgerät (1, 11) nach einem der Ansprüche 6 bis 7, wobei die Siliziumschicht die amorphe Siliziumschicht ist.

9. Prüfgerät (1, 11) nach einem der Ansprüche 6 bis 8, wobei die Schicht zur Einstellung des Brechungsindex (7, 17, 37) eine durchschnittliche Dicke von 1 nm oder mehr und 100 nm oder weniger aufweist.

10. Prüfgerät (1, 11) nach einem der Ansprüche 6 bis 9, umfassend eine Verbindung (6, 36), die in der Lage ist, durch eine Reaktion mit der Prüfsubstanz eine granulare Substanz zu erzeugen, oder eine Verbindung (56), die in der Lage ist, mit der Prüfsubstanz gebunden zu werden und eine granulare Substanz ist.

11. Prüfgerät (1, 11) nach Anspruch 10, wobei die Verbindung (6) nicht in Kontakt mit der Schicht zur Einstellung des Brechungsindex (7, 17) steht und an einer von der Schicht zur Einstellung des Brechungsindex (7, 17) entfernten Position angeordnet ist.

12. Prüfgerät (1, 11) nach Anspruch 10 oder 11, wobei die Verbindung (36) auf einer Oberfläche der Schicht zur Einstellung des Brechungsindex (37) angeordnet ist.

13. Prüfgerät (1, 11) nach einem der Ansprüche 10 bis 12, wobei

wenn das Prüfgerät (1, 11) die Verbindung (6, 36), die in der Lage ist, durch eine Reaktion mit der Prüfsubstanz eine granulare Substanz zu erzeugen, enthält, die Verbindung (6, 36), die in der Lage ist, durch eine Reaktion mit der Prüfsubstanz eine granulare Substanz zu erzeugen, eine Verbindung (6, 36) ist, die in der Lage ist, eine granulare Substanz in Aggregation durch eine Reaktion mit der Prüfsubstanz zu erzeugen, und

wenn das Prüfgerät die Verbindung (56), die in der Lage ist, mit der Prüfsubstanz gebunden zu werden und eine granulare Substanz ist, enthält, die Verbindung (56), die in der Lage ist, mit der Prüfsubstanz gebunden zu werden und eine granulare Substanz ist, eine Verbindung (56) ist, die mit der Prüfsubstanz gebunden ist und somit in der Lage ist, eine granulare Substanz in Aggregation zu erzeugen.

14. Prüfgerät (1, 11) nach einem der Ansprüche 10 bis 13, umfassend die Verbindung (56), die in der Lage ist, mit der Prüfsubstanz gebunden zu werden und eine granulare Substanz ist, wobei

die Prüfsubstanz ein Antigen enthält, und
die Verbindung (56) einen Antikörper (57) enthält.

15. Prüfgerät (1, 11) nach einem der Ansprüche 10 bis 13, umfassend die Verbindung (6, 36), die in der Lage ist, durch eine Reaktion mit der Prüfsubstanz eine granulare Substanz zu erzeugen, wobei

die Prüfsubstanz ein Glykolipid ist, und
die Verbindung (6, 36) ein Enzym ist, das mit dem Glykolipid umgesetzt werden kann.

16. Prüfgerät (1, 11) nach einem der Ansprüche 10 bis 13, umfassend die Verbindung (6, 36), die in der Lage ist, durch eine Reaktion mit der Prüfsubstanz eine granulare Substanz zu erzeugen, wobei

die Prüfsubstanz Endotoxin ist, und
die Verbindung (6, 36) ein LAL-Reagenz ist.

17. Prüfgerät (1, 11) nach einem der Ansprüche 6 bis 16, wobei die die Prüfsubstanz enthaltende Lösung eine Lösung ist, die eine biologische Probe enthält.

18. Prüfgerät (1, 11) nach einem der Ansprüche 6 bis 17, das ein Mikrochip ist.

19. Prüfvorrichtung (20, 40), umfassend:

das Prüfgerät (1, 11) nach einem der Ansprüche 6 bis 18;
eine Lichtquelle (28) zur Bestrahlung der periodischen Struktur (3A, 13A) im Prüfgerät (1, 11) mit Licht; und
eine Messvorrichtung (29) zum Messen einer Intensität des von der Lichtquelle (28) emittierten und an der periodischen Struktur (3A, 13A) reflektierten Lichts.

**Revendications**

1. Procédé d'inspection destiné à mesurer, en utilisant une solution contenant une substance de test contenant une substance de test et un liquide, une concentration de la substance de test, qui est contenue dans la solution contenant une substance de test, dans le liquide,

le procédé d'inspection utilisant un instrument d'inspection (1, 11),
l'instrument d'inspection (1, 11) incluant une paroi (3, 13, 33) qui a une structure périodique (3A, 13A, 33A) résultant d'une pluralité d'évidements (3a, 13a, 33a) ou d'une pluralité de saillies,
les évidements de la pluralité d'évidements (3a, 13a, 33a) ou les saillies de la pluralité de saillies incluant une couche de réglage d'indice de réfraction (7, 17, 37) sur des surfaces de ceux-ci,
la couche de réglage d'indice de réfraction (7, 17, 37) étant une couche de silicium,
le procédé d'inspection comportant les étapes consistant à :

mesurer une intensité lumineuse de référence en projetant de la lumière sur la structure périodique lorsque le liquide a été introduit dans l'instrument d'inspection ;
mesurer une intensité lumineuse d'évaluation en projetant de la lumière sur la structure périodique lorsque la solution contenant une substance de test a été introduite dans l'instrument d'inspection et que la substance de test et le liquide sont présents ; et
acquérir une valeur relative entre l'intensité lumineuse de référence et l'intensité lumineuse d'évaluation, et

le procédé d'inspection comparant la valeur relative obtenue lorsqu'un spécimen ayant une concentration connue de la solution de test est introduit dans l'instrument d'inspection, à la valeur relative obtenue lorsqu'un spécimen ayant une concentration inconnue de la substance de test est introduit dans l'instrument d'inspection, pour déterminer la concentration de la substance de test dans le spécimen ayant la concentration inconnue de la substance de test.

2. Procédé d'inspection selon la revendication 1, dans lequel

l'instrument d'inspection (1, 11) inclut un composé (6, 36) capable de produire une substance granulaire par une réaction avec la substance de test, ou un composé (56) qui est capable de se lier à la substance de test et est une substance granulaire, et

à l'étape consistant à mesurer l'intensité lumineuse d'évaluation, l'intensité lumineuse d'évaluation est mesurée en projetant de la lumière sur la structure périodique (3A, 13A) lorsque la solution contenant une substance de test a été introduite dans l'instrument d'inspection (1, 11) et que la substance granulaire est déposée sur la couche de réglage d'indice de réfraction (37).

3. Procédé d'inspection selon l'une quelconque des revendications 1 à 2, dans lequel la couche de silicium est une couche de silicium monocristallin, une couche de silicium polycristallin, une couche de silicium microcristallin ou une couche de silicium amorphe.

4. Procédé d'inspection selon l'une quelconque des revendications 1 à 3, dans lequel la couche de silicium est la couche de silicium amorphe.

5. Procédé d'inspection selon l'une quelconque des revendications 1 à 4, dans lequel la solution contenant une substance de test est une solution contenant un échantillon biologique.

6. Instrument d'inspection (1, 11) utilisé pour mesurer, en utilisant une solution contenant une substance de test contenant une substance de test et un liquide, une concentration de la substance de test, qui est contenue dans la solution contenant une substance de test, dans le liquide,

l'instrument d'inspection (1, 11) comportant une paroi (3, 13, 33) qui a une structure périodique (3A, 13A, 33A) résultant d'une pluralité d'évidements (3a, 13a, 33a) ou d'une pluralité de saillies,

les évidements de la pluralité d'évidements (3a, 13a, 33a) ou les saillies de la pluralité de saillies incluant une couche de réglage d'indice de réfraction (7, 17, 37) sur des surfaces de ceux-ci, et

la couche de réglage d'indice de réfraction (7, 17, 37) étant une couche de silicium.

7. Instrument d'inspection (1, 11) selon la revendication 6, dans lequel la couche de silicium est une couche de silicium monocristallin, une couche de silicium polycristallin, une couche de silicium microcristallin ou une couche de silicium amorphe.

8. Instrument d'inspection (1, 11) selon l'une quelconque des revendications 6 à 7, dans lequel la couche de silicium est la couche de silicium amorphe.

9. Instrument d'inspection (1, 11) selon l'une quelconque des revendications 6 à 8, dans lequel la couche de réglage d'indice de réfraction (7, 17, 37) a une épaisseur moyenne de 1 nm ou plus et de 100 nm ou moins.

10. Instrument d'inspection (1, 11) selon l'une quelconque des revendications 6 à 9, comportant un composé (6, 36) capable de produire une substance granulaire par une réaction avec la substance de test, ou un composé (56) qui est capable de se lier à la substance de test et qui est une substance granulaire.

11. Instrument d'inspection (1, 11) selon la revendication 10, dans lequel le composé (6) n'est pas en contact avec la couche de réglage d'indice de réfraction (7, 17) et est disposé à une position éloignée de la couche de réglage d'indice de réfraction (7, 17).

12. Instrument d'inspection (1, 11) selon la revendication 10 ou 11, dans lequel le composé (36) est disposé sur une surface de la couche de réglage d'indice de réfraction (37).

13. Instrument d'inspection (1, 11) selon l'une quelconque des revendications 10 à 12, dans lequel

lorsque l'instrument d'inspection (1, 11) comporte le composé (6, 36) capable de produire une substance granulaire par une réaction avec la substance de test, le composé (6, 36) capable de produire une substance granulaire par une réaction avec la substance de test est un composé (6, 36) capable de produire une substance granulaire en agrégation par une réaction avec la substance de test, et

lorsque l'instrument d'inspection comporte le composé (56) qui est capable de se lier à la substance de test et qui est une substance granulaire, le composé (56) qui est capable de se lier à la substance de test et qui est une substance granulaire est un composé (56) qui est lié à la substance de test et est ainsi capable de produire une substance granulaire en agrégation.

14. Instrument d'inspection (1, 11) selon l'une quelconque des revendications 10 à 13, comportant le composé (56) qui est capable de se lier à la substance de test et qui est une substance granulaire, et dans lequel

la substance de test contient un antigène, et
le composé (56) contient un anticorps (57).

15. Instrument d'inspection (1, 11) selon l'une quelconque des revendications 10 à 13, comportant le composé (6, 36) capable de produire une substance granulaire par une réaction avec la substance de test, dans lequel

la substance de test est un glycolipide, et
le composé (6, 36) est une enzyme capable de réagir avec le glycolipide.

16. Instrument d'inspection (1, 11) selon l'une quelconque des revendications 10 à 13, comportant le composé (6, 36) capable de produire une substance granulaire par une réaction avec la substance de test, dans lequel

la substance de test est une endotoxine, et
le composé (6, 36) est un réactif LAL.

17. Instrument d'inspection (1, 11) selon l'une quelconque des revendications 6 à 16, dans lequel la solution contenant une substance de test est une solution contenant un échantillon biologique.

18. Instrument d'inspection (1, 11) selon l'une quelconque des revendications 6 à 17 étant une micropuce.

19. Dispositif d'inspection (20, 40) comportant :

l'instrument d'inspection (1, 11) selon l'une quelconque des revendications 6 à 18 ;
une source lumineuse (28) pour projeter de la lumière sur la structure périodique (3A, 13A) dans l'instrument d'inspection (1, 11) ; et
un dispositif de mesure (29) pour mesurer une intensité de la lumière émise à partir de la source lumineuse (28) et réfléchie sur la structure périodique (3A, 13A).

[FIG. 1.]

[FIG. 2.]

[FIG. 3.]

[FIG. 4.]

[FIG. 5.]

[FIG. 6.]

57  7  3  56  W  57  56  3a  3A  57  56

[FIG. 7.]

1  3a  7  6  X  5  2  4  3  3A

[FIG. 8.]

L1 L2 Y 3 3a 7

[FIG. 9.]

L1 L2 Y 3 3a 7

[FIG. 10.]

L1 L2 Y 3 3a 7

[FIG. 11.]

57 56 7 3 W 57 Z1 3a 3A 57 Z1

[FIG. 12.]

57 56 7 3 W 57 Z1 3a 3A Z2 57 Z1

[FIG. 13.]

90 degrees

0 degrees

-90 degrees

[FIG. 14.]

[FIG. 15.]

[FIG. 16.]

[FIG. 17.]

[FIG. 18.]

[FIG. 19.]

[FIG. 20.]

**REFERENCES CITED IN THE DESCRIPTION**

**Patent documents cited in the description**

- JP 2007327946 A
- JP 2010032436 A
- WO 2017138595 A1
- US 2009079976 A1